(19) Europäisches Patentamt
European Patent Office
Office européen des brevets

(11) EP 3 833 657 B1

(12) **EUROPEAN PATENT SPECIFICATION**

(45) Date of publication and mention of the grant of the patent:
**27.07.2022 Bulletin 2022/30**

(21) Application number: **20749897.3**

(22) Date of filing: **31.07.2020**

(51) International Patent Classification (IPC):
***A61K 47/68*** *(2017.01)* ***C07D 213/34*** *(2006.01)*
***C07C 327/22*** *(2006.01)* ***C07D 213/59*** *(2006.01)*
***A61P 35/00*** *(2006.01)*

(52) Cooperative Patent Classification (CPC):
**A61K 47/6803; A61K 47/6849; A61K 47/6851; A61P 35/00; C07C 327/22; C07D 213/34; C07D 213/59;** C07C 2601/08; C07C 2601/16

(86) International application number:
**PCT/EP2020/071640**

(87) International publication number:
**WO 2021/019071 (04.02.2021 Gazette 2021/05)**

(54) **AMINOTHIOLESTER COMPOUNDS AND USES THEREOF**

AMINOTHIOLESTERVERBINDUNGEN UND VERWENDUNGEN DAVON

AMINOTHIOLESTERS ET LEURS UTILISATIONS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Validation States:
**MA TN**

(30) Priority: **31.07.2019 EP 19305989**

(43) Date of publication of application:
**16.06.2021 Bulletin 2021/24**

(73) Proprietor: **Advanced Biodesign**
**69800 Saint Priest (FR)**

(72) Inventors:
- **CEYLAN, Ismail**
**69800 SAINT-PRIEST (FR)**
- **MARTIN, Guillaume**
**69800 SAINT-PRIEST (FR)**
- **PEREZ, Mileidys**
**69800 SAINT-PRIEST (FR)**
- **BERROU, Axelle**
**69800 SAINT-PRIEST (FR)**

(74) Representative: **Lavoix**
**62, rue de Bonnel**
**69448 Lyon Cedex 03 (FR)**

(56) References cited:
**WO-A1-2017/064241 US-A1- 2007 032 476**

- **QUASH G ET AL: "Aldehyde dehydrogenase inhibitors: alpha,beta-Acetylenic N-substituted aminothiolesters are reversible growth inhibitors of normal epithelial but irreversible apoptogens for cancer epithelial cells from human prostate in culture", EUROPEAN JOURNAL OF MEDICINAL CHEMISTRY, vol. 43, no. 5, May 2008 (2008-05), pages 906-916, XP022639683, ISSN: 0223-5234, DOI: 10.1016/J.EJMECH.2007.06.004 [retrieved on 2008-05-01]**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 833 657 B1

## Description

The present invention relates to novel aminoesters compounds or its pharmaceutically acceptable salts or optical isomers, racemates, diastereoisomers, enantiomers or tautomers. The present invention also relates to their process of preparation and to these compounds for use as a medicament, in particular for the prevention or treatment of cancer. The present invention further relates to an antibody drug conjugate comprising such compounds.

## Background of the invention

Cancer is one of the major health problems in developed countries today. Cancer is an unregulated proliferation of cells due to loss of normal controls, resulting in unregulated growth, lack of differentiation, local tissue invasion, and, often, metastasis. Cancer can develop in any tissue or organ at any age.

Some cancers are curable if detected at an early stage, and long-term can also be possible in later stages. However, cure is not always possible and is not attempted in some advanced cases in which palliative care provides better quality of life than aggressive treatment, particularly in the elderly or in patients with underlying comorbid disorders.

Apoptosis is involved in tissue development, differentiation, and renewal. Inducing apoptosis is thus of major interest from a therapeutic viewpoint.

A very large variety of natural or synthetic anticancer medicinal products currently available are apoptosis-inducing compounds.

Among these antineoplastic medicinal products, mention may be made of alkylating agents such as cyclophosphamide, nitrosureas such as 1,3-bis(2-chloroethyl)-1-nitrosourea (BCNU), intercalating agents such as actinomycin D or adriamycin, purine or pyrimidine base analogues such as 6-thioguanine and 5-fluorouracil, inhibitors of the de novo synthesis of purine bases, such as methotrexate, and finally tubulin polymerization inhibitors such as Taxol(R).

One of the main drawbacks in using these substances is the absence of selective apoptotic activity on tumour cells.

Thus, it remains necessary to have available molecules which induce maximum apoptosis in tumour tissue while causing the least possible injury, and in a reversible manner, to the healthy tissues of the body.

QUASH G ET AL ("Aldehyde dehydrogenase inhibitors: alpha,beta-Acetylenic N-substituted aminothiolesters are reversible growth inhibitors of normal epithelial but irreversible apoptogens for cancer epithelial cells from human prostate in culture", European Journal Of Medicinal Chemistry, vol. 43, no. 5, (2008), pages 906-916) describes 4-methyl-2-pentynethioic acid S-methyl ester compounds as apoptosis inducers that find application in the treatment of cancers.

Aminothiolesters compounds and their application, notably in the treatment of cancer, are also described in US2007/0032476.

WO2017/064241 describes the use of such aminothiolesters compounds in combination with a compound able to increase $H_2O_2$ level in cancer cells of a subject.

## Description of the invention

The inventors of the present invention have identified new compounds of formula (I), which present interesting properties in the prevention or treatment of cancer.

The present invention is as defined in the claims.

It relates to a compound of formula (I):

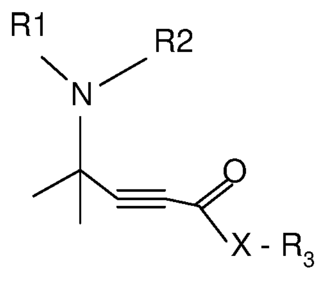
(I)

In which:

- X is S;
- R1 is linear or branched ($C_1$-$C_7$)alkyl,

- R2 is $CHR_5CHR_6OR_4$ or $(CHR_5)_vOR_4$,
- v is chosen from 2 to 4;
- $R_3$ is linear or branched $(C_1-C_7)$alkyl;
- $R_4$ is chosen from: H, linear or branched $(C_2-C_7)$alkyl, linear or branched ($C_2$-C7)alkenyl, $-CONR_7R_8$, aryl, heteroaryl, $(C_2-C_7)$cycloalkyl, linear or branched $-(C_1-C_7)$alkyl-aryl and linear or branched $-(C_1-C_7)$alkyl-heteroaryl;
  said aryl, $(C_2-C_7)$cycloalkyl, and heteroaryl being optionally substituted by one or more substituents chosen from: halogen, linear or branched $(C_1-C_7)$alkyl optionally substituted by one or more halogen atom, linear or branched $(C_1-C_7)$alkoxy optionally substituted by one or more halogen atom, -COOH, aryl, -NRR', $-NO_2$, or said aryl and heteroaryl being optionally fused to form an heterocycloalkyl;
- $R_5$ and $R_6$ are chosen from:

  - H, or
  - $R_5$ is H and R1 and $R_6$ are linked together to form with the nitrogen atom linked to R1 an heterocycloalkyl, or
  - $R_6$ is H and R1 and $R_5$ are linked together to R1 to form with the nitrogen atom linked to R1 an heterocycloalkyl;

- $R_7$ is $-(C_1-C_3)$alkyl;
- $R_8$ is $-(C_1-C_3)$alkylNRR';
- R and R' identical or different, are independently chosen from H and linear or branched $(C_1-C_7)$alkyl,
  or its pharmaceutically acceptable salts or optical isomers, racemates, diastereoisomers, enantiomers or tautomers.

In on embodiment, a compound described is a compound of formula (I) as mentioned above, in which:

- X is an atom chosen from O or S;
- R1 and R2 identical or different are independently chosen from: linear or branched $(C_1-C_7)$alkyl, linear or branched $(C_2-C_7)$alkenyl, aryl, heteroaryl, $CHR_5CHR_6OR_4$ and $(CHR_5)_vOR_4$,
  said aryl and heteroaryl being optionally substituted by one or more substituents chosen from: linear or branched $(C_1-C_7)$alkyl, halogen, $NO_2$ and $CONH_2$ ;
- v is chosen from 2 to 4;
- $R_3$ is chosen from linear or branched $(C_1-C_7)$alkyl and linear or branched $(C_1-C_7)$alkyl-$NY_1Y_2$; said linear or branched $(C_1-C_7)$alkyl-$NY_1Y_2$ being optionally substituted by $(C_1-C_7)$alkyl $-CO_2Z$;
- $R_4$ is chosen from: H, linear or branched $(C_2-C_7)$alkyl, linear or branched $(C_2-C_7)$alkenyl, aryl, heteroaryl, linear or branched $-(C_1-C_7)$alkyl-aryl and linear or branched $-(C_1-C_7)$alkyl-heteroaryl;
  said aryl and heteroaryl being optionally substituted by one or more substituents chosen from: linear or branched $(C_1-C_7)$alkyl optionally substituted by one or more halogen atom, linear or branched $(C_1-C_7)$alkoxy optionally substituted by one or more halogen atom, -COOH, aryl, -NRR', $-NO_2$ or said aryl and heteroaryl being optionally fused to form an heterocycloalkyl;
- $R_5$ and $R_6$ identical or different are independently chosen from:

  - H and linear or branched $(C_1-C_7)$alkyl, or
  - $R_5$ and $R_6$ are linked together to form with the carbon atoms to which they are attached a cycloalkyl, aryl or heteroaryl, or
  - $R_5$ is H and R1 and $R_6$ are linked together to form with the nitrogen atom linked to R1 an heterocycloalkyl or heteroaryl, or
  - $R_6$ is H and R1 and $R_5$ are linked together to R1 to form with the nitrogen atom linked to R1 an heterocycloalkyl;

- R and R' identical or different, are independently chosen from H and linear or branched $(C_1-C_7)$alkyl,
- $Y_1$ and $Y_2$ identical or different are independently chosen from H and -CO-(Ci-C7)alkyl;
- Z is chosen from H and linear or branched $(C_1-C_7)$alkyl;

  and in which, at least one of R1 and R2 is $CHR_5CHR_6OR_4$ or $(CHR_5)_vOR_4$ when X is S and R3 is linear or branched $(C_1-C_7)$alkyl;
  or its pharmaceutically acceptable salts or optical isomers, racemates, diastereoisomers, enantiomers or tautomers.

According to another embodiment, a compound according to the invention is a compound of formula (I) as mentioned above, in which R3 is methyl, R1 is methyl, R2 is $CHR_5CHR_6OR_4$ or $(CHR_5)_vOR_4$ and R5 and R6 are:

- H, or

- $R_5$ is H and R1 and $R_6$ are linked together to form with the nitrogen atom linked to R1 a pyrrolidinyl, or
- $R_6$ is H and R1 and $R_5$ are linked together to R1 to form with the nitrogen atom linked to R1 a pyrrolidinyl.

In particular, $R_4$ is chosen from H, linear or branched ($C_2$-$C_7$)alkyl, linear or branched ($C_2$-$C_7$)alkenyl, -$CONR_7R_8$, ($C_2$-$C_7$)cycloalkyl, linear or branched -($C_1$-$C_7$)alkyl-heteroaryl, aryl, or benzyl; said ($C_2$-$C_7$) cycloalkyl being substituted by one or more substituents chosen from: linear or branched (C1-$C_7$)alkyl ; said benzyl being optionally substituted by one or more substituents chosen from: linear or branched ($C_1$-$C_7$)alkyl optionally substituted by one or more halogen atom, linear or branched ($C_1$-$C_7$)alkoxy optionally substituted by one or more halogen atom, halogen , or said benzyl being optionally fused to form 1,3-benzodioxole.

Alternatively, in particular, $R_4$ is chosen from H, linear or branched ($C_2$ -$C_7$)alkyl, linear or branched ($C_2$-$C_7$)alkenyl, linear or branched -($C_1$-$C_7$)alkyl-heteroaryl, aryl, linear or branched -($C_1$-$C_7$)alkyl-aryl or benzyl; said benzyl being optionally substituted by one or more substituents chosen from: linear or branched ($C_1$-$C_7$)alkyl optionally substituted by one or more halogen atom, linear or branched ($C_1$-$C_7$)alkoxy optionally substituted by one or more halogen atom, halogen or pyridyl, or said benzyl being optionally fused to form 1,3-benzodioxole.

More particularly, $R_5$ and $R_6$ are H and $R_4$ is chosen from H, linear or branched ($C_2$ - $C_7$)alkyl, linear or branched ($C_2$-$C_7$)alkenyl, $CONR_7R_8$, ($C_2$-$C_7$)cycloalkyl, linear or branched -($C_1$-$C_7$)alkyl-heteroaryl, or benzyl; said ($C_2$-$C_7$)cycloalkyl being substituted by one or more substituents chosen from: linear or branched (C1-$C_7$)alkyl; said benzyl being optionally substituted by one or more substituents chosen from: linear or branched (C1-$C_7$)alkyl optionally substituted by one or more halogen atom, linear or branched ($C_1$-$C_7$)alkoxy optionally substituted by one or more halogen atom, halogen.

Alternatively, more particularly, $R_5$ and $R_6$ are H and $R_4$ is chosen from H, linear or branched ($C_2$ -$C_7$)alkyl, linear or branched ($C_2$-$C_7$)alkenyl, linear or branched -($C_1$-$C_7$)alkyl-heteroaryl, linear or branched -($C_1$-$C_7$)alkyl-aryl or benzyl; said benzyl being optionally substituted by one or more substituents chosen from: linear or branched (C1-$C_7$)alkyl optionally substituted by one or more halogen atom, linear or branched ($C_1$-$C_7$)alkoxy optionally substituted by one or more halogen atom, halogen.

Even more particularly, R1 is methyl and $R_4$ is chosen from: H, $CONR_7R_8$ with R7 being a methyl and $R_8$ being NRR' with R and R' being methyl, ethyl, propene, benzyl, pyridyl, benzyloxybutyl, methyl-cyclohexenyl substituted by one or more methyl, and benzyl substituted by one of more fluorine, chlorine, methoxy or methyl.

Alternatively, even more particularly, R1 is methyl and $R_4$ is chosen from: H, ethyl, propene, benzyl, pyridyl, benzyloxybutyl and benzyl substituted by one of more fluorine, chlorine, methoxy or methyl.

According to a specific embodiment, a compound of formula (I) is chosen from:

- S-methyl 4-[2-ethoxyethyl(methyl)amino]-4-methyl-pent-2-ynethioate;
- S-methyl 4-[2-allyloxyethyl(methyl)amino]-4-methyl-pent-2-ynethioate;
- S-methyl 4-[2-benzyloxyethyl(methyl)amino]-4-methyl-pent-2-ynethioate;
- S-methyl 4-methyl-4-[methyl-[2-(m-tolylmethoxy)ethyl]amino]pent-2-ynethioate;
- S-methyl 4-[2-[(3,4-dimethylphenyl)methoxy]ethyl-methyl-amino]-4-methyl-pent-2-ynethioate;
- S-methyl 4-[2-[(4-methoxyphenyl)methoxy]ethyl-methyl-amino]-4-methyl-pent-2-ynethioate;
- S-methyl 4-[2-[(3,4-dimethoxyphenyl)methoxy]ethyl-methyl-amino]-4-methyl-pent-2-ynethioate;
- S-methyl 4-[2-[(3-chlorophenyl)methoxy]ethyl-methyl-amino]-4-methyl-pent-2-ynethioate;
- S-methyl 4-[2-[(3-fluorophenyl)methoxy]ethyl-methyl-amino]-4-methyl-pent-2-ynethioate;
- S-methyl 4-methyl-4-[methyl-[2-(2-pyridylmethoxy)ethyl]amino]pent-2-ynethioate;
- S-methyl 4-methyl-4-[methyl-[2-(3-pyridylmethoxy)ethyl]amino]pent-2-ynethioate;
- S-methyl 4-methyl-4-[methyl-[2-(4-pyridylmethoxy)ethyl]amino]pent-2-ynethioate;
- 2-amino-3-((4-(dimethylamino)-4-methylpent-2ynoyl)thio)propanoïc acid;
- 2-amino-4-((4-dimethylamino)-4-methylpentyl-2-ynoyl)thio)butanoïc acid;
- ethyl-2-acetamido-3-((4-(dimethylamino)-4-methylpent-2-ynoyl)thio)propanoate;
- S-methyl 4-((4-(benzyloxy)butyl)(methyl)amino)-4-methylpent-2-ynethioate;
- S-methyl 4-((2-hydroxyethyl)(methyl)amino)-4-methylpent-2-ynethioate;
- S-methyl 4-methyl-4-[methyl-[2-(2-naphthylmethoxy)ethyl]amino]pent-2-ynethioate;
- S-methyl 4-methyl-4-[methyl-[2-[(2,6,6-trimethylcyclohexen-1-yl)methoxy]ethyl]amino]pent-2-ynethioate;
- 2-[(1,1-dimethyl-4-methylsulfanyl-4-oxo-but-2-ynyl)-methylamino] ethyl-3,4-dimethoxybenzoate;
- 2[(1,1-dimethyl-4-methylsulfanyl-4-oxo-but-2-ynyl)-methylamino] ethyl acetate;
- S-methyl 2,5,10,11,11-pentamethyl-6-oxo-7-oxa-2,5,10-triazatetradec-12-yne-14-thioate;
- S-methyl 4-[2-(methoxymethyl)pyrrolidin-1-yl]-4-methylpent-2-ynethioate;
- S-methyl 4-(3-methoxypyrrolidin-1-yl)-4-methylpent-2-ynethioate;
- S-methyl 4-methyl-4-[methyl(2-phenoxycyclopentyl)amino]pent-2-ynethioate;
- (S)-S-methyl 4-(2-((benzyloxy)methyl)pyrrolidin-1-yl)-4-methylpent-2-ynethioate;

- S-methyl 4-[(3(benzyloxy)-1pyrrolidinyl])-4-methylpent-2-ynethioate
  or its pharmaceutically acceptable salts or optical isomers, racemates, diastereoisomers, enantiomers or tautomers.

According to a specific embodiment, a compound of formula (I) is chosen from:

- S-methyl 4-[2-benzyloxyethyl(methyl)amino]-4-methyl-pent-2-ynethioate; and
- S-methyl 4-[2-[(3,4-dimethylphenyl)methoxy]ethyl-methyl-amino]-4-methyl-pent-2-ynethioate;

or its pharmaceutically acceptable salts or optical isomers, racemates, diastereoisomers, enantiomers or tautomers.
In another embodiment, a compound according to the invention is a compound of formula (I) as mentioned above, in which:

- X is S;
- R1 is linear or branched ($C_1$-$C_7$)alkyl;
- R2 is $CHR_5CHR_6OR_4$ or $(CHR_5)_vOR_4$;
- R4 is chosen from H, aryl, heteroaryl, linear or branched -($C_1$-$C_7$)alkyl-aryl and linear or branched -($C_1$-$C_7$)alkyl-heteroaryl;
  said aryl and heteroaryl being optionally substituted by one or more substituents chosen from: -COOH, -NRR' and -$NO_2$; and
- R and R' identical, are H.

The invention also relates to a process for preparing a compound of formula (I) as described herein, comprising :

a) reacting a compound of formula (II) with an organic or inorganic acid

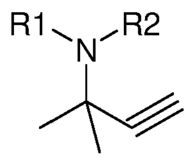

(II)

b) reacting the compound obtained in step a) with a base ;
c) reacting the compound obtained in step b) with $CO_2$ ;
d) reacting the compound obtained in step c) with alkyl chloroformate, a reagent able of forming, with the compound obtained in step c), an acid halide or a reagent able of forming, with the compound obtained in step c), a mixed anhydride ;
e) reacting the compound obtained in step d) with an anion precursor compound $SMe^-$;

wherein R1 and R2 are defined herein.
In particular, it relates to a process as mentioned above in which the base of step b) has a pKa greater than 25, preferably the base used in step b) is selected from lithium or magnesium bases, preferably the base is selected from butyllithium, or hexyllithium.
The invention also relates to a pharmaceutical composition comprising a compound of formula (I) as described herein and a pharmaceutical acceptable excipient.
It further relates to a compound of formula (I) as described herein for use as a medicament.
In particular, it relates to a compound of formula (I) as described herein for use for the prevention or treatment of cancer.
More particularly, the invention relates to a compound of formula (I) as described herein for use for the prevention or treatment of leukemia.
The invention also relates to an antibody drug conjugate of formula: B-L-Ab, wherein:

- B is a compound of formula (I) as mentioned above, in which:
- X is S;
- R1 is linear or branched ($C_1$-$C_7$)alkyl;
- R2 is $CHR_5CHR_6OR_4$ or $(CHR_5)_vOR_4$;
- R4 is chosen from H, aryl, heteroaryl, linear or branched -($C_1$-$C_7$)alkyl-aryl and linear or branched -($C_1$-$C_7$)alkyl-heteroaryl;

said aryl and heteroaryl being optionally substituted by one or more substituents chosen from: -COOH, -NRR' and $-NO_2$; and

- R and R' identical, are H;
- L is a chemical moiety comprising a covalent bond or a chain of atoms that covalently attaches the Ab to the compound of formula (I); and
- Ab is an antibody chosen from: rituximab, trastuzumab, alemtuzumab, ibritumomab, tiuxetan, tositumomab, brevacizumab, cetuximab, panitumumab, ofatumumab, ipilimumab and obinutuzumab.

A compound of formula (I) according to the invention is as above mentioned.

In one embodiment, R3 is methyl.

In another embodiment, R1 is a methyl, and R2 is $CHR_5CHR_6OR_4$ or $(CHR_5)_vOR_4$.

In one embodiment, $R_4$ is chosen from H, linear or branched ($C_2$ -$C_7$)alkyl, , linear or branched ($C_2$-$C_7$)alkenyl, linear or branched -($C_1$-$C_7$)alkyl-heteroaryl, aryl, linear or branched -($C_1$-$C_7$)alkyl-aryl or benzyl; said benzyl being optionally substituted by one or more substituents chosen from: linear or branched ($C_1$-$C_7$)alkyl optionally substituted by one or more halogen atom, linear or branched ($C_1$-$C_7$)alkoxy optionally substituted by one or more halogen atom, halogen or pyridyl, or said benzyl being optionally fused to form 1,3-benzodioxole, in particular $R_4$ is chosen from H,linear or branched ($C_2$ -$C_7$)alkyl, , linear or branched ($C_2$-$C_7$)alkenyl, linear or branched -($C_1$-$C_7$)alkyl-heteroaryl, linear or branched - ($C_1$-$C_7$)alkyl-aryl or benzyl; said benzyl being optionally substituted by one or more substituents chosen from: linear or branched ($C_1$-$C_7$)alkyl optionally substituted by one or more halogen atom, linear or branched ($C_1$-$C_7$)alkoxy optionally substituted by one or more halogen atom or halogen, and more particularly $R_4$ is chosen from: H, ethyl, propenyl, benzyl, pyridyl, benzyloxybutyl and benzyl substituted by one of more fluorine, chlorine, methoxy or methyl.

Unless specified otherwise, the terms used hereabove or hereafter as regards to the compounds of formula (I) have the meaning ascribed to them below:

- $_V$ is chosen from 2 to 4 means that the substituent "$CHR_5$" is repeated twice $CHR_5CHR_5OR_4$, three times $CHR_5CHR_5CHR_5OR_4$ or four times $CHR_5CHR_5CHR_5CHR_5OR_4$;
- "halogen" refers to fluorine, chlorine, brome or iodine atom, in particular fluorine or chlorine atom.
- "alkyl" represents an aliphatic-hydrocarbon group which may be straight or branched, having 1 to 7 or 2 to 7 carbon atoms in the chain ($C_1$-$C_7$)alkyl or ($C_2$-$C_7$)alkyl, unless specified otherwise. In particular, alkyl groups have 1 to 3 carbon atoms in the chain ($C_1$-$C_3$) alkyl. Branched means that one or more alkyl groups such as methyl, ethyl or propyl are attached to a linear alkyl chain. Exemplary alkyl groups include methyl, ethyl, n-propyl, i-propyl, n-butyl, t-butyl, 2,2-dimethylbutyl, n-pentyl, n-hexyl, n-heptyl, in particular methyl or ethyl.
- "alkenyl" refers to an aliphatic hydrocarbon group containing a carbon-carbon double bond and which may be straight or branched having 2 to 7 carbon atoms in the chain ($C_2$-$C_7$)alkenyl, unless specified otherwise. Preferred alkenyl groups have 2 to 3 carbon atoms in the chain ($C_2$-$C_3$)alkenyl. Exemplary alkenyl groups include ethenyl, n-propenyl, i-propenyl, n butenyl, i-butenyl, 2,2-dimethylbut-1-enyl, n-pentenyl, in particular propenyl.
- "alkoxy" represent an alkyl group as previously defined singular bonded to oxygen. Examples of linear or branched ($C_1$-$C_7$)alkoxy include methoxy ($CH_3O$-) and ethoxy ($CH_3CH_2O$-) .
- "aryl" refers to an aromatic monocyclic or multicyclic hydrocarbon ring system of 6 to 14 carbon atoms, preferably of 6 to 10 carbon atoms. Exemplary aryl groups include phenyl, naphthyl, benzyl, phenanthryl, biphenyl, in particular phenyl.
- "heteroaryl" refers to a 5 to 14, preferably 5 to 10 membered aromatic mono-, bi- or multicyclic ring wherein at least one member of the ring is a hetero atom. Hetero atoms can be O or N, in particular N. In particular, each ring comprises from 1 to 3 hetero atoms. Examples include pyrrolyl, pyridyl, piperidinyl, pyrazolyl, pyrimidinyl, pyrazinyl, indolyl, imidazolyl, in particular pyridyl.
- "cycloalkyl" refers to a saturated monocyclic or bicyclic non-aromatic hydrocarbon ring of 2 to 7 carbon atoms, preferably 3 to 6 carbon atoms, which can comprise one or more unsaturation. Specific examples of monocyclic cycloalkyl groups include cyclopropyl, cyclobutyl, cyclopentyl, cyclopentenyl, cyclohexyl, cyclohexenyl.

Preferably, the cycloalkyl group is cyclohexenyl.

- "-($C_1$-$C_7$)alkyl-aryl" or "-($C_1$-$C_7$)alkyl-heteroaryl" means that $R_4$ is linked to the oxygen atom by the carbon of the alkyl group; in particular -($C_1$-$C_7$)alkyl-aryl is a benzyl.
- -"heterocycle" or "heterocyclloalkyl" refers to a saturated or partially unsaturated non aromatic stable 3 to 14, preferably 5 to 10-membered mono, bi or multicyclic rings which can optionally be bridged and wherein at least one member of the ring is a hetero atom. Typically, heteroatoms include, but are not limited to O or N. In particular, each ring comprises from 1 to 3 hetero atoms. Suitable heterocycles are also disclosed in the Handbook of Chemistry and Physics, 76th Edition, CRC Press, Inc., 1995-1996, pages 225 to 226, the disclosure of which is hereby incorporated by reference. Examples of heterocycloalkyl include, but are not limited to tetrahydropyridyl, tetrahydropyranyl, pyrrolidinyl, piperidyl, morpholinyl, imidazolidinyl, or benzodioxole, in particular 1,3 benzodioxole.

- The term "substituted" refers to, unless specified otherwise, a substitution with one or more substituents, which may be identical or different, for example chosen from linear or branched ($C_1$-$C_7$)alkyl, halogen, $NO_2$ and $CONH_2$, linear or branched ($C_1$-$C_7$)alkyl substituted by one or more halogen atom, linear or branched ($C_1$-$C_7$)alkoxy, linear or branched ($C_1$-$C_7$)alkoxy substituted by one or more halogen atom, aryl, - COOH, -$COOCH_2CH_3$, -NRR', $NH_2$, NHalkyl and $N(alkyl)_2$. Examples include in particular methyl, methoxy, chlorine, fluorine, $CF_3$ and $OCF_3$.

The compounds of formula (I) as described herein can comprise one or more asymmetric carbon atoms. They can therefore exist in the form of enantiomers or diastereoisomers. These enantiomers and diastereoisomers, as well as their mixtures, including racemic mixtures, form part of the invention.

The compounds of formula (I) as described herein can be provided in the form of a free base or in the form of addition salts with acids, which also form part of the invention.

These salts are advantageously prepared with pharmaceutically acceptable acids, but salts with other acids, useful for example for the purification or for the isolation of the compounds of formula (I) as described herein, also form part of the invention.

As used herein, the expression "pharmaceutically acceptable" refers to those compounds, materials, excipients, compositions or dosage forms which are, within the scope of sound medical judgment, suitable for contact with the tissues of human beings and animals without excessive toxicity, irritation, allergic response or other problem complications commensurate with a reasonable benefit/risk ratio.

As used herein, "pharmaceutically acceptable salts" refer to derivatives of the disclosed compounds wherein the parent compound is modified by making acid or base salts thereof. The pharmaceutically acceptable salts include the conventional non-toxic salts or the quaternary ammonium salts of the parent compound formed, for example, from non-toxic inorganic or organic acids. For example, such conventional non-toxic salts include those derived from inorganic acids such as hydrochloric, hydrobromic, sulfuric, sulfamic, phosphoric, nitric and the like, including mono, di or tri-salts thereof; and the salts prepared from organic acids such as acetic, propionic, succinic, tartaric, citric, methanesulfonic, benzenesulfonic, glucoronic, glutamic, benzoic, salicylic, toluenesulfonic, oxalic, fumaric, maleic, lactic and the like. Further addition salts include ammonium salts such as tromethamine, meglumine, epolamine, etc., metal salts such as sodium, potassium, calcium, zinc or magnesium.

The pharmaceutically acceptable salts of the present invention can be synthesized from the parent compound which contains a basic or acidic moiety by conventional chemical methods. Generally, such salts can be prepared by reacting the free acid or base forms of these compounds with a stoichiometric amount of the appropriate base or acid in water or in an organic solvent, or in a mixture of the two. Generally, non-aqueous media like ether, ethyl acetate, ethanol, isopropanol, or acetonitrile are preferred. Lists of suitable salts are found in Remington's Pharmaceutical Sciences, 20th ed., Mack PublishingCompany, Easton, PA, 2000, the disclosure of which is hereby incorporated by reference.

## Process

The present invention is also concerned with the process of preparation of the compounds of formula (I) as described herein.

The compounds and process of the present invention may be prepared in a number of ways well-known to those skilled in the art. The compounds can be synthesized, for example, by application or adaptation of the methods described below, or variations thereon as appreciated by the skilled artisan. The appropriate modifications and substitutions will be readily apparent and well known or readily obtainable from the scientific literature to those skilled in the art.

It will be appreciated that the compounds of the present invention may contain one or more asymmetrically substituted carbon atoms, and may be isolated in optically active or racemic forms. Thus, all chiral, diastereomeric, racemic forms, isomeric forms of a structure are intended, unless the specific stereochemistry or isomeric form is specifically indicated. It is well-known in the art how to prepare and isolate such optically active forms.

For example, mixtures of stereoisomers may be separated by standard techniques including, but not limited to, resolution of racemic forms, normal, reverse-phase, and chiral chromatography, preferential salt formation, recrystallization, and the like, or by chiral synthesis either from chiral starting materials or by deliberate synthesis of target chiral centers.

Compounds of the present invention may be prepared by a variety of synthetic routes. The reagents and starting materials are commercially available, or readily synthesized by well-known techniques by one of ordinary skill in the arts. All substituents, unless otherwise indicated, are as previously defined.

In the reactions described hereinafter, it may be necessary to protect reactive functional groups, for example hydroxyl, amino, imino, thio or carboxy groups, where these are desired in the final product, to avoid their unwanted participation in the reactions. Conventional protecting groups may be used in accordance with standard practice, for

examples see T.W. Greene and P. G. M. Wuts in Protective Groups in Organic Chemistry, 4th ed.(2007), John Wiley & Sons Inc., 1999; J. F. W. McOmie in Protective Groups in Organic Chemistry, Plenum Press, 1973.

Some reactions may be carried out in the presence of a base. There is no particular restriction on the nature of the base to be used in this reaction, and any base conventionally used in reactions of this type may equally be used here, provided that it has no adverse effect on other parts of the molecule, and unless otherwise indicated. Examples of suitable bases include: sodium hydroxide, potassium carbonate, triethylamine, alkali metal hydrides, such as sodium hydride and potassium hydride; alkyllithium compounds, such as methyllithium and butyllithium; and alkali metal alkoxides, such as sodium methoxide and sodium ethoxide.

Usually, reactions are carried out in a suitable solvent. A variety of solvents may be used, provided that it has no adverse effect on the reaction or on the reagents involved. Examples of suitable solvents include: hydrocarbons, which may be aromatic, aliphatic or cycloaliphatic hydrocarbons, such as hexane, cyclohexane, benzene, toluene and xylene; amides, such as dimethylformamide; alcohols such as ethanol and methanol and ethers, such as diethyl ether and tetrahydrofuran.

The reactions can take place over a wide range of temperatures. In general, it is found convenient to carry out the reaction at a temperature of from 0°C to 150°C (more preferably from about room temperature to 100°C). The time required for the reaction may also vary widely, depending on many factors, notably the reaction temperature and the nature of the reagents. However, provided that the reaction is effected under the preferred conditions outlined above, a period of from 3 hours to 20 hours will usually suffice.

The compound thus prepared may be recovered from the reaction mixture by conventional means. For example, the compounds may be recovered by distilling off the solvent from the reaction mixture or, if necessary, after distilling off the solvent from the reaction mixture, pouring the residue into water followed by extraction with a water-immiscible organic solvent and distilling off the solvent from the extract. Additionally, the product can, if desired, be further purified by various well-known techniques, such as recrystallization, reprecipitation or the various chromatography techniques, notably column chromatography or preparative thin layer chromatography.

The process of preparation of a compound of formula (I) of the invention is a further object of the present invention.

According to a first aspect, a compound of the invention of formula (I) can be obtained by

a) reacting a compound of formula (II) with an organic or inorganic acid

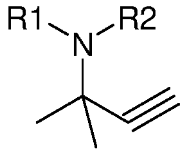

(II);

b) reacting the compound obtained in step a) with a base ;

c) reacting the compound obtained in step b) with $CO_2$ ;

d) reacting the compound obtained in step c) with alkyl chloroformate, a reagent able of forming, with the compound obtained in step c), an acid halide or a reagent able of forming, with the compound obtained in step c), a mixed anhydride ;

e) reacting the compound obtained in step d) with an anion precursor compound SMe-;

wherein R1 and R2 are as defined herein.

In particular, the base of step b) has a pKa greater than 25, preferably the base used in step b) is selected from lithium or magnesium bases, preferably the base is selected from butyllithium, or hexyllithium.

In particular, the compound of formula (II) is obtained by a step a1) of reaction between 3-chloro-3-methylbut-1-yne with R1R2NH in an aqueous medium.

In particular, said compound obtained in step a1) is purified by one or more filtrations, for example in filtration or in a succession of 2 to 10 filtrations, preferably in a succession of 2 to 5 filtrations, for example in 4 filtrations.

In one embodiment, 3-chloro-3-methylbut-1-yne is obtained by a reaction step of reacting 2-methylbut-3-yn-2-ol with hydrochloric acid in the presence of a copper catalyst.

In another embodiment, the acid is an inorganic acid chosen from hydrochloric acid, phosphoric acid, nitric acid, sulfuric acid, preferably hydrochloric acid.

In another embodiment, step d) is carried out with:

- an alkyl chloroformate having a $(C_1-C_6)$alkyl, which may comprise at least one double bond, preferably methyl, ethyl, isoprenyl, tert-butyl or isobutyl chloroformate, preferably isobutyl chloroformate; or

- a reagent capable of forming with the compound obtained in step c) a mixed anhydride chosen from acid chlorides, for example pivaloyl chloride; or
- a reagent capable of forming, with the compound obtained in step c), an acid halide chosen from $SOCl_2$, $COCl_2$, $PCl_3$, $PCl_5$, $PBr_3$ or $PPh_3$ $Br_2$.

In one embodiment, the anion precursor compounds SMe- are chosen from the salts of formula XSMe in which X represents an alkali metal or alkaline earth metal, for example Na, methyl mercaptan, or $(SMe)_2$, preferably NaSMe.
This process is described in detail in the patent applications FR 1651283 and PCT/EP2017/053457, from which the content is incorporated by reference.
Alternatively, a compound according to the invention can be prepared from the corresponding acetylenic amine treated successively by BuLi, COS and MeI. A detailed process of preparation can be found for example in G.Quash et al., European Journal of Medicinal Chemistry 43 (2008) 906-916, from which the content is incorporated by reference, in particular in the part 2 of the Material and Methods section.
The above reactions can be carried out by the skilled person by applying or adapting the methods illustrated in the examples hereinafter.
Further, the process of the invention may also comprise the additional step of isolating the compound of formula (I) or (II). This can be done by the skilled person by any of the known conventional means, such as the recovery methods described above.
Generally, the starting products are commercially available mainly from Aldrich or Acros or other typical chemicals supplier or may be obtained by applying or adapting any known methods or those described in the examples.

Use

As already mentioned, the present invention also relates to a compound of formula (I) as herein described for use as a medicament.
More particularly, it relates to a compound of formula (I) as herein described for the prevention and/or treatment of cancer.
The present invention also relates to a method of prevention and/or treatment of a cancer, comprising the administration to a subject in need thereof of an effective amount of a compound of formula (I) as described herein.
The terms "treat", "treating", "treated" or "treatment", as used in the context of the invention, refer to therapeutic treatment wherein the object is to eliminate or lessen symptoms. Beneficial or desired clinical results include, but are not limited to, elimination of symptoms, alleviation of symptoms, diminishment of extent of condition, stabilized (i.e., not worsening) state of condition, delay or slowing of progression of the condition.
The terms "prevent", "prevention", "preventing" or "prevented", as used in the context of the present invention, refer to the prevention of the onset, recurrence or spread of a disease or disorder, or of one or more symptoms thereof. In certain embodiments, the terms refer to the treatment with or administration of a compound provided herein prior to the onset of symptoms, particularly to patients at risk of disease or disorders provided herein. The terms encompass the inhibition or reduction of a symptom of the particular disease. Subjects with familial history of a disease in particular are candidates for preventive regimens in certain embodiments. In addition, subjects who have a history of recurring symptoms are also potential candidates for the prevention. In this regard, the term "prevention" may be interchangeably used with the term "prophylactic treatment".
As used herein and unless otherwise defined, "cancer" refers to the growth, division or proliferation of abnormal cells in the body. It refers to any type of malignant (i.e. non benign) tumor. The malignant tumor may correspond to a primary tumor or to a secondary tumor (i.e. a metastasis). Further, the tumor may correspond to a solid malignant tumor, which includes e.g. carcinomas, adenocarcinomas, sarcomas, melanomas, mesotheliomas, blastomas, or to a blood cancer such as leukemias, lymphomas and myelomas. The cancer may for example correspond to a solid carcinoma, a melanoma, a lung cancer (including but not limited to non-small cell lung carcinomas (NSCLC), small cell lung carcinoma (SCLC), combined small cell carcinomas, pleuropulmonary blastomas, carcinoid tumors, sarcomatoid carcinomas, carcinoid tumors, adenosquamous carcinomas, squamous cell lung carcinomas, adenocarcinomas and large cell lung carcinomas), a brain cancer (including but not limited to gliomas, glioblastomas, astrocytomas, oligoastrocytomas, oligodendrogliomas and ependymomas), kidney cancer, prostate cancer, breast cancer, myelodysplastic syndrome and leukemia.
In particular, the present invention relates to a compound of formula (I) as herein described for the prevention and/or treatment of leukemia.
In particular, the subject in need of a treatment against cancer is a subject afflicted with such disease.
In the context of the present invention, the identification of the subjects who are in need of treatment of herein-described diseases and conditions is conducted as above mentioned and is well within the ability and knowledge of the man skilled in the art. A clinician skilled in the art can readily identify, by the above mentioned technics, those subjects

who are in need of such treatment.

A therapeutically effective amount can be readily determined by the attending diagnostician, as one skilled in the art, by the use of conventional techniques and by observing results obtained under analogous circumstances. In determining the therapeutically effective amount, a number of factors are considered by the attending diagnostician, including, but not limited to: the species of subject; its size, age, and general health; the specific disease involved; the degree of involvement or the severity of the disease; the response of the individual subject; the particular compound administered; the mode of administration; the bioavailability characteristic of the preparation administered; the dose regimen selected; the use of concomitant medication; and other relevant circumstances.

As used herein, an «effective amount" refers to an amount which is effective in reducing, eliminating, treating or controlling the symptoms of the herein-described diseases and conditions. The term "controlling" is intended to refer to all processes wherein there may be a slowing, interrupting, arresting, or stopping of the progression of the diseases and conditions described herein, but does not necessarily indicate a total elimination of all disease and condition symptoms, and is intended to include prophylactic treatment and chronic use.

The term "patient" or "subject" refers to a warm-blooded animal such as a mammal, in particular a human, male or female, unless otherwise specified, which is afflicted with, or has the potential to be afflicted with one or more diseases and conditions described herein.

The amount of the compound according to the invention, which is required to achieve the desired biological effect, will vary depending upon a number of factors, including the dosage of the drug to be administered, the chemical characteristics (e.g. hydrophobicity) of the compounds employed, the potency of the compounds, the type of disease, the diseased state of the patient, and the route of administration.

Compounds provided herein can be formulated into pharmaceutical compositions, optionally by admixture with one or more pharmaceutically acceptable excipients.

Such compositions may be prepared for use in oral administration, particularly in the form of tablets or capsules, in particular orodispersible (lyoc) tablets; or parenteral administration, particularly in the form of liquid solutions, suspensions or emulsions.

It may be prepared by any of the methods well known in the pharmaceutical art, for example, as described in Remington: The Science and Practice of Pharmacy, 20th ed.; Gennaro, A. R., Ed.; Lippincott Williams & Wilkins: Philadelphia, PA, 2000. Pharmaceutically compatible binding agents and/or adjuvant materials can be included as part of the composition. Oral compositions will generally include an inert diluent carrier or an edible carrier. They can be administered in unit dose forms, wherein the term "unit dose" means a single dose which is capable of being administered to a patient, and which can be readily handled and packaged, remaining as a physically and chemically stable unit dose comprising either the active compound itself, or as a pharmaceutically acceptable composition.

The tablets, pills, powders, capsules, troches and the like can contain one or more of any of the following ingredients, or compounds of a similar nature: a binder such as microcrystalline cellulose, or gum tragacanth; a diluent such as starch or lactose; a disintegrant such as starch and cellulose derivatives; a lubricant such as magnesium stearate; a glidant such as colloidal silicon dioxide; a sweetening agent such as sucrose or saccharin; or a flavoring agent such as peppermint, or methyl salicylate. Capsules can be in the form of a hard capsule or soft capsule, which are generally made from gelatin blends optionally blended with plasticizers, as well as a starch capsule. In addition, dosage unit forms can contain various other materials that modify the physical form of the dosage unit, for example, coatings of sugar, shellac, or enteric agents. Other oral dosage forms syrup or elixir may contain sweetening agents, preservatives, dyes, colorings, and flavorings. In addition, the active compounds may be incorporated into fast dissolve, modified-release or sustained-release preparations and formulations, and wherein such sustained-release formulations are preferably bimodal.

Liquid preparations for administration include sterile aqueous or non-aqueous solutions, suspensions, and emulsions. The liquid compositions may also include binders, buffers, preservatives, chelating agents, sweetening, flavoring and coloring agents, and the like. Non-aqueous solvents include alcohols, propylene glycol, polyethylene glycol, acrylate copolymers, vegetable oils such as olive oil, and organic esters such as ethyl oleate. Aqueous carriers include mixtures of alcohols and water, hydrogels, buffered media, and saline. In particular, biocompatible, biodegradable lactide polymer, lactide/glycolide copolymer, or polyoxyethylene-polyoxypropylene copolymers may be useful excipients to control the release of the active compounds. Intravenous vehicles can include fluid and nutrient replenishers, electrolyte replenishers, such as those based on Ringer's dextrose, and the like.

Examples of modes of administration include parenteral e.g. subcutaneous, intramuscular, intravenous, intradermal, as well as oral administration.

## Antibody drug conjugate

As already mentioned, the present invention also relates to an antibody drug conjugate of formula: B-L-Ab, wherein:

- B is a compound of formula (I) as mentioned above, in which:
- X is S;
- R1 is linear or branched ($C_1$-$C_7$)alkyl;
- R2 is $CHR_5CHR_6OR_4$ or $(CHR_5)_vOR_4$ ;
- R4 is chosen from H, aryl, heteroaryl, linear or branched -($C_1$-$C_7$)alkyl-aryl and linear or branched -($C_1$-$C_7$)alkyl-heteroaryl;
  said aryl and heteroaryl being optionally substituted by one or more substituents chosen from: -COOH, -NRR' and -$NO_2$; and
- R and R' identical, are H;
- L is a chemical moiety comprising a covalent bond or a chain of atoms that covalently attaches the Ab to the compound of formula (I); and
- Ab is an antibody chosen from: rituximab, trastuzumab, alemtuzumab, ibritumomab tiuxetan, tositumomab, brevacizumab, cetuximab, panitumumab, ofatumumab, ipilimumab and obinutuzumab.

By "antibody drug conjugate" or ADC is meant an important class of highly potent biopharmaceutical drugs designed as a targeted therapy for the treatment of people with cancer. Unlike chemotherapy, ADCs are intended to target and kill only the cancer cells and spare healthy cells. ADCs are molecules composed of an antibody linked to a biologically active cytotoxic (anticancer) payload or drug. In the context of the present invention, the cytotoxic drug is the compound of formula (I) mentioned above. This compound is linked to the antibody by a linker.

"Linker" as used herein, means a chemical moiety comprising a covalent bond or a chain of atoms that covalently attaches the antibody to the compound of formula (I) as mentioned above.

The linker of the antibody drug conjugate can be any linker able to conjugate the antibody and the above-mentioned compound of formula (I). Suitable linking groups are well known in the art. In particular, it can be a biodegrabable linker.

More particularly, the compound according to the invention as above mentioned, is coupled to antibody via an attachment group (maleimide, succinimidyl ester, specific peptidic sequence substrate of enzyme, etc ...), linked to a cleavable linker (protease site, hydrazine, disulfide) or a non-cleavable linker and with or not a self-imolative spacer.

In the above definition of the antibody drug conjugate B-L-Ab, the linker L thus includes both the linker and eventually the linker linked to an attachment group as defined herein.

Cleavable dipeptide linkers like *Val-Ala* and *Val-Cit* can be cited as examples. They take advantage of the antibody-drug conjugate targeting mechanism which involves sequential binding of the antibody-drug conjugate to its cognate antigen on the surface of the target cancer cells, and internalization of the ADC-antigen complexes through the endosomal-lysosomal pathway.

In these cases, intracellular release of the cytotoxic anticancer drug relies on the fact that endosomes/lysosomes are *acidic compartments* that will facilitate cleavage of *acid-labile chemical linkages* such as *hydrazone.* In addition, if a lysosomal-specific protease cleavage site is engineered into the linker, for example the *cathepsin B* site in *vcMMAE,* the cytotoxins will be liberated in proximity to their intracellular targets.

Alternatively, linkers containing mixed disulfides provide yet another approach by which cytotoxic payloads can be liberated intracellularly as they are selectively cleaved in the reducing environment of the cell, but not in the oxygen-rich environment in the bloodstream.

These linkers can be prepared by methods well known by the man skill in the art.

In particular, the linker according to the invention is the maleimidocaproyl-Val-Cit described in the experimental part.

Other examples of linkers that can be used in the context of the invention as well as methods of preparation thereof can be Maleimidocaproyl linker, Mercaptoacetamidocaproyl, Hydrazone linker and Glucuronide combined with a self-immolative linker p-aminobenzyl alcohol (PAB) as mentioned in Perez et al.: "Antibody-drug conjugates: current status and future directions"; Drug Discovery Today, Volume 00, Number 00, December 2013 and in McCombs and Shawn: "Antibody Drug Conjugates: Design and Selection of Linker, Payload and Conjugation Chemistry", The AAPS Journal, Vol.17, No.2, March 2015.

The antibodies are commercially available and well known by the man skilled in the art.

More information regarding these antibodies is given in the table 1 below.

| Year | International non-proprietary name/Trade name | Target | Indication |
|---|---|---|---|
| 1997 | Rituximab/Rituxan® | CD20 | B-cell lymphoma |
| 1998 | Trastuzumab/Herceptin® | HER2 | Breast cancer |
| 2001 | Alemtuzumab/Campath® | CD52 | Chronic lymphocytic leukemia |

(continued)

| Year | International non-proprietary name/Trade name | Target | Indication |
|---|---|---|---|
| 2002 | Ibritumomab tiuxetan/Zevalin® | CD20 | B-cell lymphoma |
| 2003 | Tositumomab/Bexxar® | CD20 | B-cell lymphoma |
| 2004 | Bevacizu m ab/Avasti n® | VEGF | Colon, lung, breast and renal cancer |
| 2004 | Cetuximab/Erbitux® | EGFR | Colon; lung cancer |
| 2004 | Gemtuzumab/MYLOTARG® | CD33 | Acute Myeloid Leukemia |
| 2006 | Panitumumab/Vectibix® | EGFR | Colon cancer |
| 2009 | Ofatumumab/Arzerra® | CD20 | Chronic lymphocytic leukemia |
| 2013 | Obinutuzumab/ Gazyvaro® | CD20 | Chronic lymphocytic leukemia |

The method to prepare the antibody drug conjugate according to the invention will be adapted by the man skilled in the art in function of the linker and the antibody chosen. The man skilled in the art will be able to prepare the antibody drug conjugate on the basis of its general knowledge. An exemple is given in the experimental part of the present invention.

The present invention also relates to the antibody drug conjugate as defined above for use as a medicament, in particular for use for the prevention and/or treatment of cancer.

Examples of cancer to treat are B-cell lymphoma, breast cancer, chronic lymphocytic leukemia, colon cancer, lung cancer, breast cancer, renal cancer and melanoma.

The present invention further relates to a pharmaceutical composition comprising an antibody drug conjugate according to the invention.

The terms "treat", "treating", "treated" or "treatment", as used in the context of the invention, refer to therapeutic treatment wherein the object is to eliminate or lessen symptoms. Beneficial or desired clinical results include, but are not limited to, elimination of symptoms, alleviation of symptoms, diminishment of extent of condition, stabilized (i.e., not worsening) state of condition, delay or slowing of progression of the condition.

The terms "prevent", "prevention", "preventing" or "prevented", as used in the context of the present invention, refer to the prevention of the onset, recurrence or spread of a disease or disorder, or of one or more symptoms thereof. In certain embodiments, the terms refer to the treatment with or administration of a compound provided herein prior to the onset of symptoms, particularly to patients at risk of disease or disorders provided herein. The terms encompass the inhibition or reduction of a symptom of the particular disease. Subjects with familial history of a disease in particular are candidates for preventive regimens in certain embodiments. In addition, subjects who have a history of recurring symptoms are also potential candidates for the prevention. In this regard, the term "prevention" may be interchangeably used with the term "prophylactic treatment".

As used herein and unless otherwise defined, "cancer" refers to the growth, division or proliferation of abnormal cells in the body. It refers to any type of malignant (i.e. non benign) tumor. The malignant tumor may correspond to a primary tumor or to a secondary tumor (i.e. a metastasis).

In particular, the subject in need of a treatment against cancer is a subject afflicted with such disease.

In the context of the present invention, the identification of the subjects who are in need of treatment of herein-described diseases and conditions is conducted as above mentioned and is well within the ability and knowledge of the man skilled in the art. A clinician skilled in the art can readily identify, by the above-mentioned technics, those subjects who are in need of such treatment.

A therapeutically effective amount can be readily determined by the attending diagnostician, as one skilled in the art, by the use of conventional techniques and by observing results obtained under analogous circumstances. In determining the therapeutically effective amount, a number of factors are considered by the attending diagnostician, including, but not limited to: the species of subject; its size, age, and general health; the specific disease involved; the degree of involvement or the severity of the disease; the response of the individual subject; the particular compound administered; the mode of administration; the bioavailability characteristic of the preparation administered; the dose regimen selected; the use of concomitant medication; and other relevant circumstances.

As used herein, an «effective amount" refers to an amount which is effective in reducing, eliminating, treating or controlling the symptoms of the herein-described diseases and conditions. The term "controlling" is intended to refer to all processes wherein there may be a slowing, interrupting, arresting, or stopping of the progression of the diseases and conditions described herein, but does not necessarily indicate a total elimination of all disease and condition symptoms, and is intended to include prophylactic treatment and chronic use.

The term "patient" or "subject" refers to a warm-blooded animal such as a mammal, in particular a human, male or female, unless otherwise specified, which is afflicted with, or has the potential to be afflicted with one or more diseases and conditions described herein.

The amount of the antibody drug conjugate according to the invention, which is required to achieve the desired biological effect, will vary depending upon a number of factors, including the dosage to be administered, the chemical and biological characteristics (e.g. hydrophobicity) of the compounds employed, the potency of the compounds, the type of disease, the diseased state of the patient, and the route of administration.

Antibody drug conjugate provided herein can be formulated into pharmaceutical compositions, optionally by admixture with one or more pharmaceutically acceptable excipients.

Such compositions may be prepared for use in oral administration, particularly in the form of tablets or capsules, in particular orodispersible (lyoc) tablets; or parenteral administration, particularly in the form of liquid solutions, suspensions or emulsions.

It may be prepared by any of the methods well known in the pharmaceutical art, for example, as described in Remington: The Science and Practice of Pharmacy, 20th ed.; Gennaro, A. R., Ed.; Lippincott Williams & Wilkins: Philadelphia, PA, 2000. Pharmaceutically compatible binding agents and/or adjuvant materials can be included as part of the composition. Oral compositions will generally include an inert diluent carrier or an edible carrier. They can be administered in unit dose forms, wherein the term "unit dose" means a single dose which is capable of being administered to a patient, and which can be readily handled and packaged, remaining as a physically and chemically stable unit dose comprising either the active compound itself, or as a pharmaceutically acceptable composition.

The tablets, pills, powders, capsules, troches and the like can contain one or more of any of the following ingredients, or compounds of a similar nature: a binder such as microcrystalline cellulose, or gum tragacanth; a diluent such as starch or lactose; a disintegrant such as starch and cellulose derivatives; a lubricant such as magnesium stearate; a glidant such as colloidal silicon dioxide; a sweetening agent such as sucrose or saccharin; or a flavoring agent such as peppermint, or methyl salicylate. Capsules can be in the form of a hard capsule or soft capsule, which are generally made from gelatin blends optionally blended with plasticizers, as well as a starch capsule. In addition, dosage unit forms can contain various other materials that modify the physical form of the dosage unit, for example, coatings of sugar, shellac, or enteric agents. Other oral dosage forms syrup or elixir may contain sweetening agents, preservatives, dyes, colorings, and flavorings. In addition, the active compounds may be incorporated into fast dissolve, modified-release or sustained-release preparations and formulations, and wherein such sustained-release formulations are preferably bi-modal.

Liquid preparations for administration include sterile aqueous or non-aqueous solutions, suspensions, and emulsions. The liquid compositions may also include binders, buffers, preservatives, chelating agents, sweetening, flavoring and coloring agents, and the like. Non-aqueous solvents include alcohols, propylene glycol, polyethylene glycol, acrylate copolymers, vegetable oils such as olive oil, and organic esters such as ethyl oleate. Aqueous carriers include mixtures of alcohols and water, hydrogels, buffered media, and saline. In particular, biocompatible, biodegradable lactide polymer, lactide/glycolide copolymer, or polyoxyethylene-polyoxypropylene copolymers may be useful excipients to control the release of the active compounds. Intravenous vehicles can include fluid and nutrient replenishers, electrolyte replenishers, such as those based on Ringer's dextrose, and the like.

Examples of modes of administration include parenteral e.g. subcutaneous, intramuscular, intravenous, intradermal, as well as oral administration.

In the scope of the present invention, it has to be understood that "a compound for use in the treatment or prevention of" is equivalent to "the use of a compound for the treatment or prevention of" and to "the use of a compound for the manufacture of a medicament intended for the treatment or prevention of".

The invention will be further illustrated by the following figure and examples.

## FIGURE

**Figure 1:** Mean viability of Raji cells in percentage comparing to the non-treated cells, after a treatment of 50$\mu$g/ml of Rituximab (85.50 % $\pm$ 2.268 %) and Rituximab (102.9 % $\pm$ 1.789 %) coupled with compound 5. Difference between the two means was significantly using Unpaired t-test (P < 0.01, **).

## EXAMPLES

Representative compounds of the invention (except compounds 13, 14, 15 and 16 which are not part of the invention), are summarized in the table 2 below:

**Table 2**

| Example | Structure | Name |
|---|---|---|
| 1 | 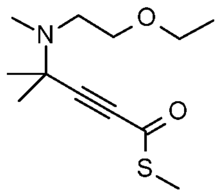 | S-methyl 4-[2-ethoxyethyl(methyl)amino]-4-methyl-pent-2-yneth ioate |
| 2 | 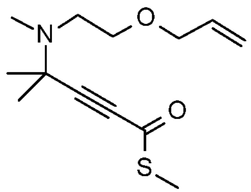 | S-methyl 4-[2-allyloxyethyl(methyl)amino]-4-methyl-pent-2-yneth ioate |
| 3 | 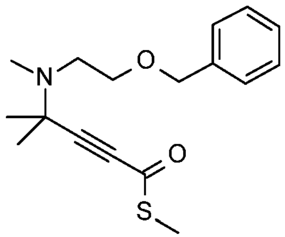 | S-methyl 4-[2-benzyloxyethyl(methyl)amino]-4-methyl-pent-2-yneth ioate |
| 4 | 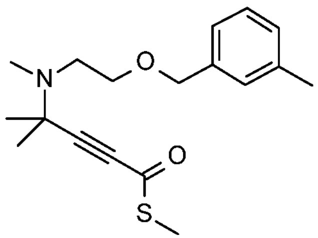 | S-methyl 4-methyl-4-[methyl-[2-(m-tolylmethoxy)ethyl]amino]pent -2-ynethioate |

(continued)

| Example | Structure | Name |
|---|---|---|
| 5 | 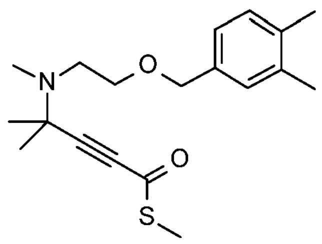 | S-methyl 4-[2-[(3,4-dimethylphenyl)methoxy]ethylmethyl-amino]-4-methyl-pent-2-ynethioate |
| 6 | 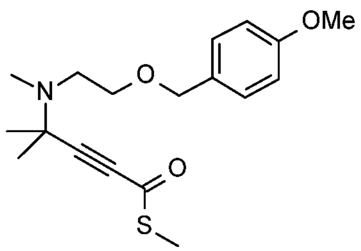 | S-methyl 4-[2-[(4-methoxyphenyl)methoxy]ethylmethyl-amino]-4-methyl-pent -2-ynethioate |
| 7 | 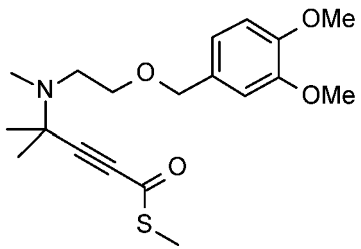 | S-methyl 4-[2-[(3,4-dimethoxyphenyl)methoxy]ethylmethyl-amino]-4-methyl-pent -2-ynethioate |
| 8 | 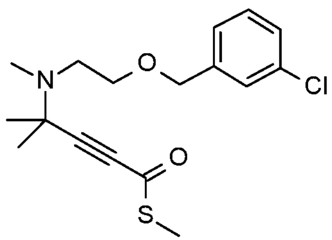 | S-methyl 4-[2-[(3-chlorophenyl)meth oxy]ethylmethyl-amino]-4-methyl-pent -2-ynethioate |

(continued)

| Example | Structure | Name |
|---|---|---|
| 9 | 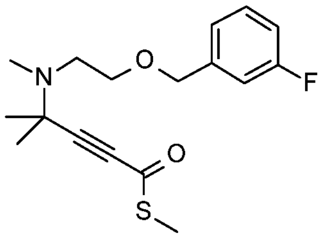 | S-methyl 4-[2-[(3-fluorophenyl)methoxy]ethyl-methyl-amino]-4-methyl-pent-2-ynethioate |
| 10 | 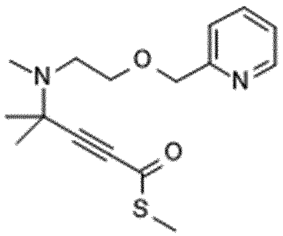 | S-methyl 4-methyl-4-[methyl-[2-(2-pyridylmethoxy)ethyl]amino]pent -2-ynethioate |
| 11 | 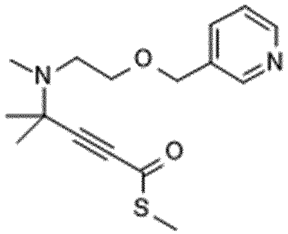 | S-methyl 4-methyl-4-[methyl-[2-(3-pyridylmethoxy)ethyl]amino]pent -2-ynethioate |
| 12 | 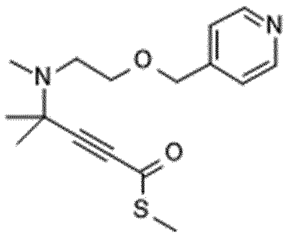 | S-methyl 4-methyl-4-[methyl-[2-(4-pyridylmethoxy)ethyl]amino]pent -2-ynethioate |

(continued)

| Example | Structure | Name |
|---|---|---|
| 13 | 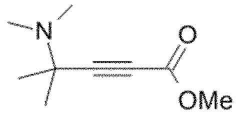 | methyl 4-(dimethylamino)-4-methyl-pent-2-ynoate (is not part of the invention) |
| 14 | 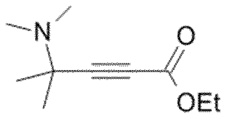 | ethyl 4-(dimethylamino)-4-methyl-pent-2-ynoate (is not part of the invention) |
| 15 | 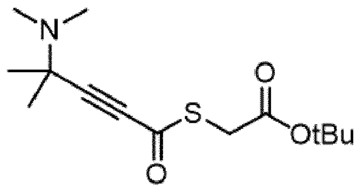 | tert-butyl 2-((4-(dimethylamino)-4-methylpent-2-ynoyl)thio)acetate (is not part of the invention) |
| 16 | 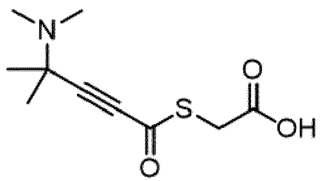 | 2-((4-(dimethylamino)-4-methylpent-2-ynoyl)thio)acetic acid (is not part of the invention) |
| 17 | 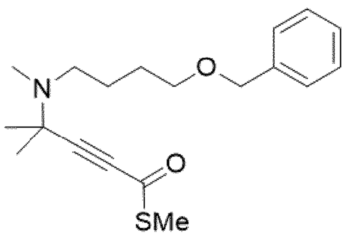 | S-methyl 4-((4-(benzyloxy)butyl)(methyl)amino)-4-methylpent-2-ynethioate |

(continued)

| Example | Structure | Name |
|---|---|---|
| 18 | 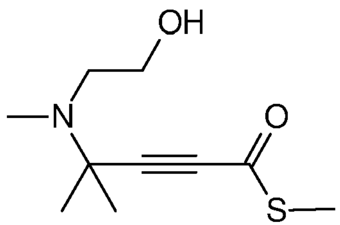 | S-methyl 4-((2-hydroxyethyl)(methyl)amino)-4-methylpent-2-ynethioate |
| 19 | 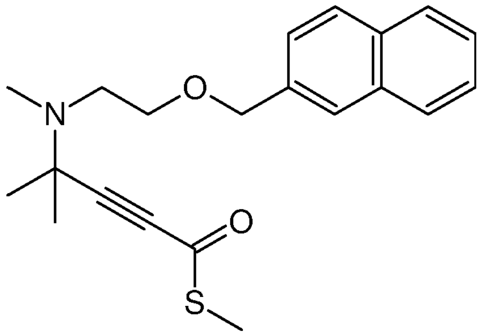 | S-methyl 4-methyl-4-[methyl-[2-(2-naphthylmethoxy)ethyl]amino]pent-2-ynethioate |
| 20 | 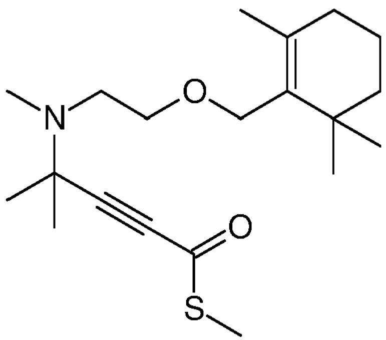 | S-methyl 4-methyl-4-[methyl-[2-[(2,6,6-trimethylcyclohexen-1-yl)methoxy]ethyl]amino]pent -2-ynethioate |

(continued)

| Example | Structure | Name |
|---|---|---|
| 21 | 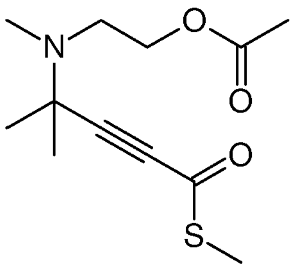 | 2-[(1,1-dimethyl-4-methylsulfanyl-4-oxo-but-2-ynyl)-methylamino] ethyl acetate |
| 22 | 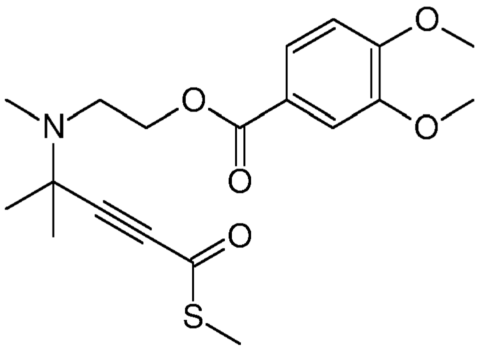 | 2-[(1,1-dimethyl-4-methylsulfanyl-4-oxo-but-2-ynyl)-methylamino] ethyl-3,4-dimethoxybenzoate |
| 23 | 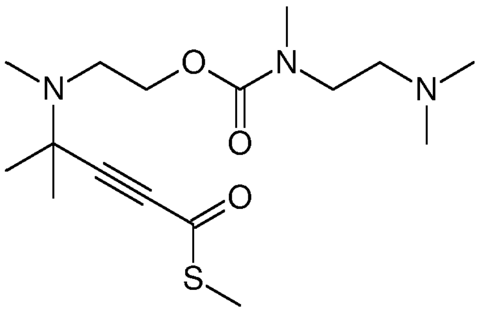 | S-methyl 2,5,10,11,11-pentamethyl-6-oxo-7-oxa-2,5,10-triazatetradec-12-yne-14-thioate |

(continued)

| Example | Structure | Name |
|---|---|---|
| 24 | 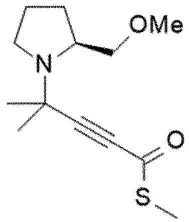 | S-methyl 4-[2-(methoxymethyl)pyrrolidin-1-yl]-4-methylpent-2-ynethioate |
| 25 | 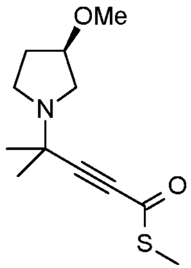 | S-methyl 4-(3-methoxypyrrolidin-1-yl)-4-methylpent-2-ynethioate |
| 26 | 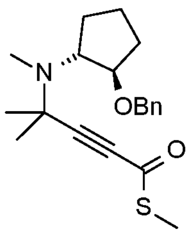 | S-methyl 4-methyl-4-[methyl(2-phenoxycyclopentyl)amino]pent-2-ynethioate |
| 27 | 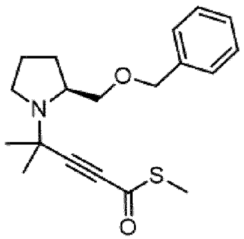 | (S)-S-methyl 4-(2-((benzyloxy)methyl)pyrrolidin-1-yl)-4-methylpent-2-ynethioate |

(continued)

| Example | Structure | Name |
|---|---|---|
| 28 | 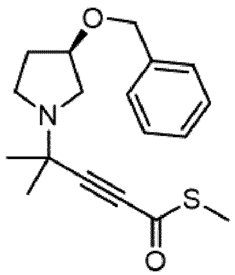 | S-methyl 4-[(3(benzyloxy)-1pyrrolidinyl])-4-methylpent-2-ynethioate |
| 29 | 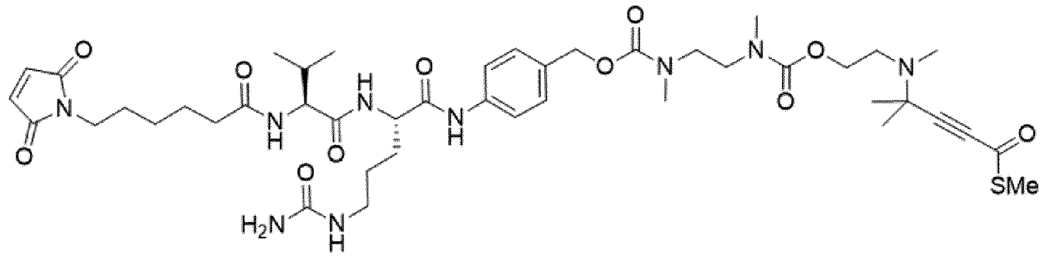 | |

Representative compounds of the invention (except compounds 13, 14, 15 and 16 which are not part of the invention) can be synthesized according to the following procedures.

***General analytical procedures***

The $^{1}H$ and $^{13}C$ NMR spectra were recorded on a Bruker Advance ALS300 and DRX400 MHz from Bruker. Chemical shifts are reported in ppm ($\delta$) and were referenced to DMSO-*d6* ($^{1}H$, 2.50 ppm; $^{13}C$, 39.52 ppm) or CDCl3 (7.26 ppm). The coupling constants (J) were given in Hz.

The HRMS-ESI mass spectra were recorded in positive-ion mode on a hybrid quadrupole time-of-flight mass spectrometer (MicroTOFQ-II, Bruker Daltonics, Bremen) with an Electrospray Ionization (ESI) ion source. For the mass spectrometry of low resolution, LRMS-ESI mass spectra were recorded in a Thermo Finnigan MAT 95 XL spectrometer.

**Part 1: Preparation of the compounds according to the invention**

**Example 1: S-methyl 4-[2-ethoxyethyl(methyl)amino]-4-methyl-pent-2-ynethioate**

**Preparation of N-(2-ethoxyethyl)-N,2-dimethyl-but-3-yn-2-amine** : To a solution of N-methyl-N-(2'hydroxyethyl)-3-amino-3methyl-1-butyne (Easton, Nelson R.; Hennion, George F. U.S. (1967), US 3337625 19670822.) (1.0 g, 7.08 mmol) and iodoethane (0.98 mL, 7.6 mmol) in THF (12 mL) was added NaH (0.459 g, 11.5 mmol) at room temperature and the mixture was refluxed for 3 h. Mixture was then carefully hydrolyzed at room temperature by water and extracted by EtOAc (3x25 mL). Combined organic layers were washed with brine, dried over $Na_2SO_4$ and concentrated in vacuo. Purification of the crude by chromatography on silicagel (petroleum ether/EtOAc=70/30) gave pure N-(2-ethoxyethyl)-N,2-dimethyl-but-3-yn-2-amine (0.479 g, 40%) .

$^{1}H$ NMR (300 MHz, DMSO) $\delta$ 3.45 - 3.36 (m, 4H), 3.11 (s, 1H), 2.51 (t, J = 6.7 Hz,. 2H), 2.20 (s, 3H), 1.27 (s, 6H), 1.09 (t, J = 7.0 Hz, 3H).

ESI-LRMS 170.0 [M+H]+.

**Preparation of S-methyl 4-[2-ethoxyethyl(methyl)amino]-4-methyl-pent-2-ynethioate :** To N-(2-ethoxyethyl)-N,2-dimethyl-but-3-yn-2-amine (0.367 g, 2.17 mmol) in THF (11 mL) was added dropwise a 2.28 M n-BuLi solution in hexane (1.14 mL, 2.60 mmol) at -70°C. After 5 min at -70°C the reaction mixture was warmed to 0°C, maintained 10 min at this temperature then cooled at -70°C before a 30 min bubbling with carbonyl sulfide (COS) through the solution. The yellow solution was warmed to 0°C, stirred for additional 10 min at this temperature before dropwise addition of iodomethane (0.162 mL, 2.60 mmol). The mixture was stirred for 2 h, carefully hydrolyzed at 0°C by water and extracted with ether. Combined organic layers were washed with brine, dried over $Na_2SO_4$ and concentrated in vacuo. Purification of the crude by chromatography on silicagel (petroleum ether/EtOAc=90/10) gave pure S-methyl 4-[2-ethoxyethyl(methyl)amino]-4-methyl-pent-2-ynethioate (0.369 g, 70%) as an near colorless oil.

$^{1}H$ NMR (300 MHz, DMSO) $\delta$ 3.42 (t, *J* = 6.3 Hz, 2H), 3.42 (q, *J* = 7.0 Hz, 2H), 2.56 (t, *J*= 6.3 Hz, 2H), 2.39 (s, 3H), 2.25 (s, 3H), 1.36 (s, 6H), 1.10 (t, *J* = 7.0 Hz, 3H).

ESI- HRMS calc for $C_{12}H_{22}NO_2S$ [M+H]+: 244.1366, found: 244.1362.

**Example 2: S-methyl 4-[2-allyloxyethyl(methyl)amino]-4-methyl-pent-2-ynethioate**

**Preparation of N-(2-allyloxyethyl)-N,2-dimethyl-but-3-yn-2-amine** : To N-methyl-N-(2'hydroxyethyl)-3-amino-3methyl-1-butyne (Easton, Nelson R.; Hennion, George F. U.S. (1967), US 3337625 19670822.)) (1.0 g, 7.08 mmol) in THF (12 mL) was added NaH (0.340 g, 8.50 mmol) at 0°C. After 15 min at 0°C and 15 min at room temperature, *n*-$Bu_4NI$ (0.026 g, 0.071 mmol) was added in one portion at 0°C followed by dropwise addition of allyl bromide (0.735 mL, 8.50 mmol). Reaction mixture was allowed to reach room temperature, stirred overnight, then carefully hydrolyzed by water and extracted by ether (3x25 mL). Combined organic layers were washed with brine (25 mL), dried over $Na_2SO_4$ and concentrated in vacuo. Purification by chromatography on silicagel (petroleum ether/ether=80/20 to 70/30) gave pure N-(2-allyloxyethyl)-N,2-dimethyl-but-3-yn-2-amine (0.941 g, 73%) as an oil.

$^{1}H$ NMR (300 MHz, DMSO) $\delta$ 5.88 (ddt, J = 17.3, 10.5, 5.3 Hz, 1H), 5.24 (ddd, J = 17.3, 3.8, 1.7 Hz, 1 H), 5.16 - 5.09 (m, 1H), 3.93 (dt, J = 5.3, 1.6 Hz, 2H), 3.43 (t, J = 6.4 Hz, 2H), 3.12 (s, 1H), 2.55 (t, J = 6.4 Hz, 2H), 2.21 (s, 3H), 1.27 (s, 6H).

ESI-LRMS 182.0 [M+H]+.

**Preparation of S-methyl 4-[2-allyloxyethyl(methyl)amino]-4-methyl-pent-2-ynethioate:** The compound is obtained by using the same process as the one described for S-methyl 4-[2-ethoxyethyl(methyl)amino]-4-methyl-pent-2-ynethioate [example 1] starting from N-(2-allyloxyethyl)-N,2-dimethyl-but-3-yn-2-amine. Scale: 2.2 mmol. Purification by chromatography on silicagel (petroleum ether/EtOAc=90/10 to 80/20). Yield : 65%. Near colorless oil.

$^{1}H$ NMR (300 MHz, DMSO) $\delta$ 5.88 (ddt, *J* = 17.3, 10.5, 5.3 Hz, 1H), 5.24 (ddd, *J* = 17.3, 3.8, 1.7 Hz, 1H), 5.17

- 5.10 (m, 1H), 3.94 (dt, *J* = 5.3, 1.5 Hz, 2H), 3.45 (t, *J* = 6.2 Hz, 2H), 2.58 (t, J= 6.2 Hz, 2H), 2.39 (s, 3H), 2.26 (s, 3H), 1.36 (s, 6H).

ESI- HRMS calc for $C_{13}H_{22}NO_2S$ [M+H]+: 256.1366, found: 256.1364.

**Example 3: S-methyl 4-[2-benzyloxyethyl(methyl)amino]-4-methyl-pent-2-ynethioate**

**Preparation of N-(2-benzyloxyethyl)-N,2-dimethyl-but-3-yn-2-amine** : The compound is obtained by using the same process as the one described for N-(2-allyloxyethyl)-N,2-dimethyl-but-3-yn-2-amine [example 2] using 1.015 eq of NaH and 1.01 eq. of benzyl bromide. Purification by chromatography on silicagel (petroleum ether/EtOAc=90/10). Yield : 81%. Colorless oil.

$^1H$ NMR (300 MHz, DMSO) $\delta$ 7.39 - 7.24 (m, 5H), 4.47 (s, 2H), 3.49 (t, J = 6.3 Hz, 2H), 3.12 (s, 1H), 2.58 (t, J = 6.3 Hz, 2H), 2.21 (s, 3H), 1.27 (s, 6H). ESI-LRMS 232.0 [M+H]+.

**Preparation of S-methyl 4-[2-benzyloxyethyl(methyl)amino]-4-methyl-pent-2-ynethioate** : The compound is obtained by using the same process as the one described for S-methyl 4-[2-ethoxyethyl(methyl)amino]-4-methyl-pent-2-ynethioate [example 1] starting from N-(2-benzyloxyethyl)-N,2-dimethyl-but-3-yn-2-amine. Scale: 2.2 mmol. Purification by chromatography on silicagel (petroleum ether/EtOAc=90/10). Yield : 79%. Colorless oil.

$^1H$ NMR (300 MHz, DMSO) $\delta$ 7.37 - 7.26 (m, 5H), 4.48 (s, 2H), 3.52 (t, *J* = 6.1 Hz, 2H), 2.62 (t, *J* = 6.1 Hz, 2H), 2.38 (s, 3H), 2.26 (s, 3H), 1.36 (s, 6H).

ESI- HRMS calc for $C_{17}H_{24}NO_2S$ [M+H]+: 306.1522, found: 306.1514.

*Alternative protocol*: To N-(2-benzyloxyethyl)-N,2-dimethyl-but-3-yn-2-amine (0.650 g, 2.81 mmol) in THF (8 mL) was added dropwise a 2.28 M n-BuLi solution in hexane (1.36 mL, 3.09 mmol) at -70°C. After 5 min at -70°C the reaction mixture was warmed to 0°C, maintained 30 min at this temperature and $CO_2$ was bubbled through the solution for 30 min. The mixture was warmed to room temperature within 5 min then re-cooled at 0°C. Isobutyl chloroformate (0.40 ml, 3.08 mmol) was added dropwise and the mixture stirred for 10 min before addition of sodium methoxide (0.236 g, 3.37 mmol) in one portion. The mixture was warmed to room temperature stirred for additional 15 min at this temperature then carefully hydrolyzed at 0°C by water and extracted with ether. Combined organic layers were washed with brine, dried over $Na_2SO_4$ and concentrated in vacuo. Purification of the crude by chromatography on silicagel (petroleum ether/EtOAc=90/10) gave pure S-methyl 4-[2-ethoxyethyl(methyl)amino]-4-methyl-pent-2-ynethioate (0.307 g, 36%).

**Example 4: S-methyl 4-methyl-4-[methyl-[2-(m-tolylmethoxy)ethyl]amino]pent-2-ynethioate**

**Preparation of N-2-dimethyl-N-[2-(m-tolylmethoxy)ethyl]but-3-yn-2-amine:** The compound is obtained by using the same process as the one described for N-(2-allyloxyethyl)-N,2-dimethyl-but-3-yn-2-amine [example 2] using 3-Methylbenzyl bromide. Purification by chromatography on silicagel (petroleum ether/EtOAc=90/10). Scale: 4.5 mmol. Yield : 79%. Colorless oil.

$^1H$ NMR (300 MHz, DMSO) $\delta$ 7.26 - 7.19 (m, 1H), 7.16 - 7.05 (m, 3H), 4.43 (s, 2H), 3.48 (t, J = 6.3 Hz, 2H), 3.12 (s, 1H), 2.57 (t, J = 6.3 Hz, 2H), 2.30 (s, 3H), 2.21 (s, 3H), 1.27 (s, 6H).

ESI-LRMS 246.1 [M+H]+.

**Preparation of S-methyl 4-methyl-4-[methyl-[2-(m-tolylmethoxy)ethyl]amino]pent-2-ynethioate** : The compound is obtained by using the same process as the one described for S-methyl 4-[2-ethoxyethyl(methyl)amino]-4-methyl-pent-2-ynethioate [example 1] starting from N-2-dimethyl-N-[2-(m-tolylmethoxy)ethyl]but-3-yn-2-amine. Scale : 1.3 mmol. Purification by chromatography on silicagel (petroleum ether/EtOAc=90/10). Yield : 77%. Colorless oil.

$^1H$ NMR (300 MHz, DMSO) $\delta$ 7.26 - 7.19 (m, 1H), 7.16 - 7.05 (m, 3H), 4.44 (s, 2H), 3.50 (t, *J* = 6.1 Hz, 2H), 2.62 (t, *J* = 6.1 Hz, 2H), 2.38 (s, 3H), 2.30 (s, 3H), 2.26 (s, 3H), 1.36 (s, 6H).

ESI- HRMS calc for $C_{18}H_{26}NO_2S$ [M+H]+: 320.1679, found: 320.1667.

**Example 5: S-methyl 4-[2-[(3,4-dimethylphenyl)methoxy]ethyl-methyl-amino]-4-methyl-pent-2-ynethioate**

**Preparation of N-[2-[(3,4-dimethylphenyl)methoxy]ethyl]-N,2-dimethyl-but-3-yn-2-amine** : The compound is obtained by using the same process as the one described for N-(2-allyloxyethyl)-N,2-dimethyl-but-3-yn-2-amine [example 2] using 3,4-Dimethylbenzyl bromide. Purification by chromatography on silicagel (petroleum ether/EtOAc=60/40). Scale : 3.8 mmol. Yield : 60%. Colorless oil.

$^1H$ NMR (300 MHz, DMSO) $\delta$ 7.12 - 7.06 (m, 2H), 7.04 - 6.99 (m, 1H), 4.39 (s, 2H), 3.45 (t, J = 6.3 Hz, 2H), 3.12 (s, 1H), 2.56 (t, J = 6.3 Hz, 2H), 2.20 (s, 6H), 2.19 (s, 3H), 1.27 (s, 6H).

ESI-LRMS 182.0 [M+H]+. ESI-LRMS 260.0 [M+H]+.

**Preparation of S-methyl 4-[2-[(3,4-dimethylphenyl)methoxy]ethyl-methyl-amino]-4-methyl-pent-2-ynethioate** : The compound is obtained by using the same process as the one described for S-methyl 4-[2-ethoxyethyl(methyl)amino]-4-methyl-pent-2-ynethioate [example 1] starting from N-[2-[(3,4-dimethylphenyl)methoxy]ethyl]-N,2-dimethyl-but-3-yn-2-amine. Scale: 1.3 mmol. Purification by chromatography on silicagel (petroleum ether/EtOAc=90/10). Yield : 77%. Near colorless oil.

$^1H$ NMR (300 MHz, DMSO) δ 7.12 - 7.06 (m, 2H), 7.05 - 6.99 (m, 1H), 4.40 (s, 2H), 3.48 (t, *J* = 6.2 Hz, 2H), 2.60 (t, *J* = 6.2 Hz, 2H), 2.38 (s, 3H), 2.25 (s, 3H), 2.20 (s, 3H), 2.19 (s, 3H), 1.36 (s, 6H).

ESI- HRMS calc for $C_{19}H_{28}NO_2S$ [M+H]+: 334.1835, found: 334.1825.

**Example 6: S-methyl 4-[2-[(4-methoxyphenyl)methoxy]ethyl-methyl-amino]-4-methyl-pent-2-ynethioate**

**Preparation of N-[2-[(4-methoxyphenyl)methoxy]ethyl]-N,2-dimethyl-but-3-yn-2-amine:** The compound is obtained by using the same process as the one described for N-(2-allyloxyethyl)-N,2-dimethyl-but-3-yn-2-amine [example 2] using 1-(Bromomethyl)-4-methoxybenzene. Purification by chromatography on silicagel (DCM/MeOH=99/1 to 97.5/2.5). Scale : 4.0 mmol. Yield : 53%. Colorless oil.

1H NMR (300 MHz, DMSO) δ 7.27 - 7.20 (m, 2H), 6.93 - 6.86 (m, 2H), 4.39 (s, 2H), 3.74 (s, 3H), 3.45 (t, J = 6.3 Hz, 2H), 3.12 (s, 1H), 2.55 (t, J = 6.4 Hz, 2H), 2.20 (s, 3H), 1.27 (s, 6H).

ESI-LRMS 261.9 [M+H]+.

**Preparation of S-methyl 4-[2-[(4-methoxyphenyl)methoxy]ethyl-methyl-amino]-4-methyl-pent-2-ynethioate:** The compound is obtained by using the same process as the one described for S-methyl 4-[2-ethoxyethyl(methyl)amino]-4-methyl-pent-2-ynethioate [example 1] starting from N-[2-[(4-methoxyphenyl)methoxy]ethyl]-N,2-dimethyl-but-3-yn-2-amine. Scale: 1.3 mmol. Purification by chromatography on silicagel (petroleum ether/EtOAc=80/20). Yield : 74%. Near colorless oil.

$^1H$ NMR (300 MHz, DMSO) δ 7.27 - 7.21 (m, 2H), 6.93 - 6.87 (m, 2H), 4.40 (s, 2H), 3.74 (s, 3H), 3.48 (t, *J* = 6.2 Hz, 2H), 2.60 (t, *J* = 6.2 Hz, 2H), 2.38 (s, 3H), 2.25 (s, 3H), 1.36 (s, 6H).

ESI- HRMS calc for $C_{18}H_{26}NO_3S$ [M+H]+: 336.1628, found: 336.1613.

**Example 7: S-methyl 4-[2-[(3,4-dimethoxyphenyl)methoxy]ethyl-methyl-amino]-4-methyl-pent-2-ynethioate**

**Preparation of N-[2-[(3,4-dimethoxyphenyl)methoxy]ethyl]-N,2-dimethyl-but-3-yn-2-amine:** The compound is obtained by using the same process as the one described for N-(2-allyloxyethyl)-N,2-dimethyl-but-3-yn-2-amine [example 2] using 4-(Bromomethyl)-1,2-dimethoxybenzene. Purification by chromatography on silicagel (DCM/MeOH=99/1 to 97.5/2.5). Scale : 4.0 mmol. Yield : 67%. Colorless oil.

$^1H$ NMR (300 MHz, DMSO) δ 6.94 - 6.88 (m, 2H), 6.87 - 6.80 (m, 1H), 4.39 (s, 2H), 3.74 (s, 3H), 3.73 (s, 3H), 3.46 (t, J = 6.3 Hz, 2H), 3.12 (s, 1H), 2.56 (t, J = 6.3 Hz, 2H), 2.21 (s, 3H), 1.27 (s, 6H).

ESI-LRMS 292.0 [M+H]+.

**Preparation of S-methyl 4-[2-[(3,4-dimethoxyphenyl)methoxy]ethyl-methyl-amino]-4-methyl-pent-2-ynethioate:** The compound is obtained by using the same process as the one described for S-methyl 4-[2-ethoxyethyl(methyl)amino]-4-methyl-pent-2-ynethioate [example 1] starting from N-[2-[(3,4-dimethoxyphenyl)methoxy]ethyl]-N,2-dimethyl-but-3-yn-2-amine. Scale: 1.0 mmol. Purification by chromatography on silicagel (petroleum ether/EtOAc=90/10). Yield : 67%. Near colorless oil.

$^1H$ NMR (300 MHz, DMSO) δ 6.94 - 6.87 (m, 1H), 6.87 - 6.81 (m, 1H), 4.40 (s, 2H), 3.74 (s, 3H), 3.73 (s, 3H), 3.48 (t, *J* = 6.1 Hz, 2H), 2.61 (t, *J* = 6.2 Hz, 2H), 2.38 (s, 3H), 2.26 (s, 3H), 1.36 (s, 6H).

ESI- HRMS calc for $C_{19}H_{28}NO_4S$ [M+H]+: 366.1734, found: 336.1720.

**Example 8: S-methyl 4-[2-[(3-chlorophenyl)methoxy]ethyl-methyl-amino]-4-methyl-pent-2-ynethioate**

**Preparation of N-[2-[(3-chlorophenyl)methoxy]ethyl]-N,2-dimethyl-but-3-yn-2-amine:** The compound is obtained by using the same process as the one described for N-(2-allyloxyethyl)-N,2-dimethyl-but-3-yn-2-amine [example 2] using 3-Chlorobenzyl bromide. Purification by chromatography on silicagel (petroleum ether/EtOAc=70/30). Scale : 4.0 mmol. Yield : 71%. Colorless oil.

$^1H$ NMR (300 MHz, DMSO) δ 7.43 - 7.25 (m, 4H), 4.49 (s, 2H), 3.50 (t, J = 6.2 Hz, 2H), 3.12 (s, 1H), 2.58 (t, J = 6.2 Hz, 2H), 2.22 (s, 3H), 1.28 (s, 6H).

ESI-LRMS 266.0 [M+H]+.

**Preparation of S-methyl 4-[2-[(3-chlorophenyl)methoxy]ethyl-methyl-amino]-4-methyl-pent-2-ynethioate:** The compound is obtained by using the same process as the one described for S-methyl 4-[2-ethoxyethyl(methyl)amino]-4-methyl-pent-2-ynethioate [example 1] starting from N-[2-[(3-chlorophenyl)methoxy]ethyl]-N,2-dimethyl-but-3-yn-2-amine except that the reaction mixture was maintained at -70°C after n-BuLi addition for 30 min before COS bubbling. Scale : 1.3 mmol. Purification by chromatography on silicagel (petroleum ether/EtOAc=75/25). Yield : 63%. Near colorless oil.

$^1H$ NMR (300 MHz, DMSO) δ 7.44 - 7.24 (m, 4H), 4.50 (s, 2H), 3.52 (t, *J* = 6.0 Hz, 2H), 2.63 (t, *J* = 6.0 Hz, 2H),

2.38 (s, 3H), 2.27 (s, 3H), 1.37 (s, 6H).

ESI- HRMS calc for $C_{17}H_{23}ClNO_2S$ [M+H]+: 340.1133, found: 340.1120.

**Example 9: S-methyl 4-[2-[(3-fluorophenyl)methoxy]ethyl-methyl-amino]-4-methyl-pent-2-ynethioate**

**Preparation of N-[2-[(3-fluorophenyl)methoxy]ethyl]-N,2-dimethyl-but-3-yn-2-amine :** The compound is obtained by using the same process as the one described for N-(2-allyloxyethyl)-N,2-dimethyl-but-3-yn-2-amine [example 2] using 3-fluorobenzyl bromide. Purification by chromatography on silicagel (petroleum ether/EtOAc=80/20). Scale : 4.0 mmol. Yield : 71%. Colorless oil.

**Preparation of S-methyl 4-[2-[(3-fluorophenyl)methoxy]ethyl-methyl-amino]-4-methyl-pent-2-ynethioate:** The compound is obtained by using the same process as the one described for S-methyl 4-[2-ethoxyethyl(methyl)amino]-4-methyl-pent-2-ynethioate [example 1] starting from N-[2-[(3-fluorophenyl)methoxy]ethyl]-N,2-dimethyl-but-3-yn-2-amine except that the reaction mixture was maintained at -70°C after n-BuLi addition for 30 min before COS bubbling. Scale : 1.3 mmol. Purification by chromatography on silicagel (petroleum ether/EtOAc=70/30). Yield : 87%. Near colorless oil.

$^1$H NMR (300 MHz, DMSO) δ 7.44 - 7.34 (m, 1H), 7.20 - 7.05 (m, 3H), 4.51 (s, 2H), 3.53 (t, *J* = 6.1 Hz, 2H), 2.63 (t, *J* = 6.1 Hz, 2H), 2.38 (s, 3H), 2.27 (s, 3H), 1.37 (s, 6H).

ESI- HRMS calc for $C_{17}H_{23}FNO_2S$ [M+H]+: 324.1428, found: 324.1415

**Example 10: S-methyl 4-methyl-4-[methyl-[2-(2-pyridylmethoxy)ethyl]amino]pent-2-ynethioate**

**Preparation of N-2-dimethyl-N-[2-(2-pyridylmethoxy)ethyl]but-3-yn-2-amine:** The compound is obtained by using the same process as the one described for N-(2-allyloxyethyl)-N,2-dimethyl-but-3-yn-2-amine [example 2] starting from 2-(Bromomethyl)pyridine hydrobromide and using 4 eq of NaH. Purification by chromatography on silicagel (DCM/MeOH=99/1 to 95/5). Scale : 2.1 mmol. Yield : 71%. Yellow oil.

$^1$H NMR (300 MHz, DMSO) δ 8.50 (ddd, J = 4.8, 1.8, 0.9 Hz, 1H), 7.80 (td, J = 7.7, 1.8 Hz, 1H), 7.44 (d, J = 7.8 Hz, 1H), 7.32 - 7.24 (m, 1H), 4.55 (s, 2H), 3.56 (t, J = 6.2 Hz, 2H), 3.13 (s, 1H), 2.61 (t, J = 6.2 Hz, 2H), 2.23 (s, 3H), 1.28 (s, 6H).

ESI-LRMS 233.1 [M+H]+.

**Preparation of S-methyl 4-methyl-4-[methyl-[2-(2-pyridylmethoxy)ethyl]amino]pent-2-ynethioate:** The compound is obtained by using the same processas the one described for S-methyl 4-[2-ethoxyethyl(methyl)amino]-4-methyl-pent-2-ynethioate [example 1] starting from N-2-dimethyl-N-[2-(2-pyridylmethoxy)ethyl]but-3-yn-2-amine and using 1.5 eq of n-BuLi,1.5 eq of MeI and DCM extractions. Scale: 0.9 mmol. Purification by chromatography on silicagel (DCM/MeOH=99/1 to 90/10). Yield : 18%. Yellow oil.

$^1$H NMR (300 MHz, DMSO) δ 8.54 - 8.48 (m, 1H), 7.80 (td, J = 7.7, 1.8 Hz, 1H), 7.44 (d, J = 7.8 Hz, 1H), 7.28 (dd, J = 6.7, 5.1 Hz, 1H), 4.56 (s, 2H), 3.59 (t, J = 6.1 Hz, 2H), 2.65 (t, J = 6.1 Hz, 2H), 2.38 (s, 3H), 2.28 (s, 3H), 1.37 (s, 6H). ).

ESI- HRMS calc for $C_{16}H_{23}N_2O_2S$ [M+H]+: 3071475, found: 307.1471.

**Example 11: S-methyl 4-methyl-4-[methyl-[2-(3-pyridylmethoxy)ethyl]amino]pent-2-ynethioate**

**Preparation of N-2-dimethyl-N-[2-(3-pyridylmethoxy)ethyl]but-3-yn-2-amine:** The compound is obtained by using the same process as the one described for N-(2-allyloxyethyl)-N,2-dimethyl-but-3-yn-2-amine [example 2] starting from 3-(Bromomethyl)pyridine hydrobromide and using 4 eq of NaH. Purification by chromatography on silicagel (DCM/MeOH=99/1 to 95/5). Scale : 2.1 mmol. Yield : 67%. Yellow oil.

$^1$H NMR (300 MHz, DMSO) δ 8.56 - 8.52 (m, 1H), 8.49 (dd, J = 4.8, 1.7 Hz, 1H), 7.78 - 7.70 (m, 1H), 7.38 (ddd, *J* = 7.8, 4.8, 0.8 Hz, 1H), 4.52 (s, 2H), 3.52 (t, *J* = 6.2 Hz, 2H), 3.12 (s, 1H), 2.58 (t, *J* = 6.2 Hz, 2H), 2.21 (s, 3H), 1.27 (s, 6H).

ESI-LRMS 233.1 $[M+H]^+$.

**Preparation of S-methyl 4-methyl-4-[methyl-[2-(3-pyridylmethoxy)ethyl]amino]pent-2-ynethioate:** The compound is obtained by using the same process as the one described for S-methyl 4-[2-ethoxyethyl(methyl)amino]-4-methyl-pent-2-ynethioate [example 1] starting from N-2-dimethyl-N-[2-(3-pyridylmethoxy)ethyl]but-3-yn-2-amine and using 1.5 eq of n-BuLi,1.5 eq of MeI and DCM extractions. Scale: 0.4 mmol. Purification by chromatography on silicagel (DCM/MeOH=99/1 to 95/5). Yield : 15%. Yellow oil.

$^1$H NMR (300 MHz, DMSO) δ 8.54 (d, J = 1.5 Hz, 1H), 8.49 (dd, J = 4.8, 1.7 Hz, 1H), 7.78 - 7.70 (m, 1H), 7.38 (ddd, J = 7.8, 4.8, 0.8 Hz, 1H), 4.53 (s, 2H), 3.54 (t, J = 6.1 Hz, 2H), 2.63 (t, J = 6.1 Hz, 2H), 2.38 (s, 3H), 2.26 (s, 3H), 1.36 (s, 6H)).

ESI- HRMS calc for $C_{16}H_{23}N_2O_2S$ [M+H]+: 3071475, found: 307.1474.

**Example 12: S-methyl 4-methyl-4-[methyl-[2-(4-pyridylmethoxy)ethyl]amino]pent-2-ynethioate**

**Preparation of N-2-dimethyl-N-[2-(4-pyridylmethoxy)ethyl]but-3-yn-2-amine:** The compound is obtained by using the same process as the one described for N-(2-allyloxyethyl)-N,2-dimethyl-but-3-yn-2-amine [example 2] starting from 4-(Bromomethyl)pyridine hydrobromide and using 4 eq of NaH. Purification by chromatography on silicagel (DCM/MeOH=99/1 to 95/5). Scale : 2.1 mmol. Yield : 95%. Yellow oil.

$^1H$ NMR (300 MHz, DMSO) δ 8.56 - 8.49 (m, 2H), 7.38 - 7.27 (m, 2H), 4.54 (s, 2H), 3.53 (t, J = 6.2 Hz, 2H), 3.13 (s, 1H), 2.61 (t, J = 6.2 Hz, 2H), 2.23 (s, 3H), 1.28 (s, 6H). ESI-LRMS 233.1 [M+H]+.

**Preparation of S-methyl 4-methyl-4-[methyl-[2-(4-pyridylmethoxy)ethyl]amino]pent-2-ynethioate:** The compound is obtained by using the same process as the one described for S-methyl 4-[2-ethoxyethyl(methyl)amino]-4-methyl-pent-2-ynethioate [example 1] starting from N-2-dimethyl-N-[2-(4-pyridylmethoxy)ethyl]but-3-yn-2-amine and using 1.5 eq of n-BuLi,1.5 eq of MeI and DCM extractions. Scale: 1.0 mmol. Purification by chromatography on silicagel (DCM/MeOH=99/1 to 95/15). Yield : 24%. Yellow oil.

$^1H$ NMR (300 MHz, DMSO) δ 8.60 - 8.45 (m, 2H), 7.37 - 7.26 (m, 2H), 4.55 (s, 2H), 3.56 (t, J = 6.0 Hz, 2H), 2.65 (t, J = 6.0 Hz, 2H), 2.38 (s, 3H), 2.28 (s, 3H), 1.37 (s, 6H).

ESI- HRMS calc for $C_{16}H_{23}N_2O_2S$ [M+H]+: 3071475, found: 307.1470.

**Example 13: methyl 4-(dimethylamino)-4-methyl-pent-2-ynoate (is not part of the invention)**

**Preparation of 4-(dimethylamino)-4-methyl-pent-2-ynoic acid chlorhydrate:** To N,N,2-trimethylbut-3-yn-2-amine (0.928 g, 8.35 mmol) in THF (42 mL) was added dropwise a 2.35 M n-BuLi solution in hexane (3.73 mL, 8.76 mmol) at -70°C. After 5 min at -70°C the reaction mixture was warmed to 0°C, maintained 10 min at this temperature then cooled at -70°C before a 45 min bubbling with carbon dioxide. The mixture was warmed to 0°C within 2 h, then carefully hydrolyzed at 0°C by water and washed (2x25 mL) with ether. Aqueous layers were acidified (PH1-2) with 6N HCl then concentrated in vacuo. The solid obtained was triturated and washed twice with MeOH. The crude 4-(dimethylamino)-4-methyl-pent-2-ynoic acid chlorhydrate (0.721 g, 45%) obtained as a white solid was used in the next step without purification.

$^1H$ NMR (300 MHz, $D_2O$) 2.94 (s, 6H), 1.70 (s, 6H). $^{13}C$ NMR (75 MHz, $D_2O$) δ 158.97 (C), 83.64 (C), 76.05 (C), 60.35 (C), 38.46 ($2CH_3$), 23.66 ($2CH_3$).

**Preparation of Methyl 4-(dimethylamino)-4-methyl-pent-2-ynoate:** 4-(dimethylamino)-4-methyl-pent-2-ynoic acid chlorhydrate (0.500 g, 2.61 mmol) in MeOH (10 mL) was treated with conc.$H_2SO_4$ (0.15 mL) at 0°C then stirred overnight at room temperature. After concentration in vacuo the residue was diluted in AcOEt. Organic layer was washed with $NaHCO_3$ aq.sat. and brine, dried over $Na_2SO_4$ and solvent evaporated in vacuo to give (yield <10%, not optimized) the methyl ester as an near colorless oil.

$^1H$ NMR (300 MHz, DMSO) δ 3.71 (s, 3H), 2.19 (s, 6H), 1.35 (s, 6H).

ESI-LRMS [M+H]+ calc for $C_9H_{15}NO_2$ [M+H]+: 170.11, found: 170.1.

**Example 14: ethyl 4-(dimethylamino)-4-methyl-pent-2-ynoate (is not part of the invention):**

The compound is obtained by using the same process as the one described in example 13 using EtOH in the esterification step. Scale 5.22 mmol. Yield: 73%. Colorless oil.

$^1H$ NMR (300 MHz, DMSO) δ 4.17 (q, *J* = 7.1 Hz, 2H), 2.19 (s, 6H), 1.34 (s, 6H), 1.22 (t, *J* = 7.1 Hz, 3H).

ESI- HRMS calc for $C_{10}H_{18}NO_2$ [M+H]+: 184.1332, found: 184.1326.

**Example 15: tert-butyl 2-((4-(dimethylamino)-4-methylpent-2-ynoyl)thio)acetate (is**

**not part of the invention):** The compound was obtained by using the same process as the one described for S-methyl 4-[2-ethoxyethyl(methyl)amino]-4-methyl-pent-2-ynethioate [example 1] starting from N,N,2-trimethylbut-3-yn-2-amine and using tert-butyl iodoacetate instead of iodomethane. Purification by chromatography on silicagel (cyclohexane/EtOAc= 70/30). Scale 3 mmol Yield : 57%. Red oil.

$^1H$ NMR (300MHz, DMSO) δ 3.82 (s, 2H), 2.21 (s, 6H), 1.41 (s, 9H), 1.37 (s, 6H).

ESI - HRMS : calc for $C_{14}H_{24}NO_3S$ [M+H]+ 286.1471, found 286.1472.

**Example 16: 2-((4-(dimethylamino)-4-methylpent-2-ynoyl)thio)acetic acid (is not part of the invention)**

To a solution of tert-butyl 2-((4-(dimethylamino)-4-methylpent-2-ynoyl)thio)acetate (200 mg, 0.7 mmol) in dichloromethane (3.6 mL) is added trifluoroacetic acid (0.36 mL). The mixture is stirred overnight in the dark. After evaporation under reduced pressure the crude was triturated and washed with $Et_2O$. The TFA salt was obtained as an amorphous

solid. Yield : 84%.

1H NMR (300MHz, acetone) δ 12.14 (s, 1H), 3.95 (s, 2H), 2.99 (s, 6H), 1.87 (s, 6H).

ESI - HRMS : calc for $C_{10}H_{16}NO_3S$ [M+H]+ 230.0845, found 230.0847

**Example 17: S-methyl 4-((4-(benzyloxy)butyl)(methyl)amino)-4-methylpent-2-ynethioate**

**Preparation of 4-(methyl(2-methylbut-3-yn-2-yl)amino)butan-1-ol** : This compound was prepared by standard protocols previously described for the synthesis of N-methyl-N-(2'hydroxyethyl)-3-amino-3methyl-1-butyne (Easton, Nelson R.; Hennion, George F. U.S. (1967), US 3337625 19670822.) starting from commercially available 4-(methylamino)butan-1-ol. 4-(methyl(2-methylbut-3-yn-2-yl)amino)butan-1-ol was obtained as a bright yellow oil. Scale 3 mmol. Yield : 99%.

$^1$H NMR (300MHz, DMSO) δ 4.41 (t, J = 5.2Hz, 1H), 3.42 - 3.33 (m, 2H), 3.09 (s, 1H), 2.38 - 2.29 (m, 2H), 2.14 (s, 3H), 1.45 - 1.36 (m, 4H), 1.27 (s, 6H).

ESI - LRMS : 170.1 [M+H]+

**Preparation of N-(4-(benzyloxy)butyl)-N,2-dimethylbut-3-yn-2-amine** : The compound was obtained by using the same process as the one described for N-(2-allyloxyethyl)-N,2-dimethyl-but-3-yn-2-amine [example 2] starting from 4-(methyl(2-methylbut-3-yn-2-yl)amino)butan-1-ol and using 1.015 eq of NaH and 1.01 eq. of benzyl bromide. Purification by chromatography on silicagel (cyclohexane/EtOAc=80/20). Scale 2.4 mmol. Yield : 55%. Yellow oil.

$^1$H NMR (300MHz, DMSO) δ 7.39 - 7.22 (m, 5H), 4.44 (s, 2H), 3.42 (t, J = 6.3Hz, 2H), 3.08 (s, 1H), 2.34 (t, J = 6.9Hz, 2H), 2.13 (s, 3H), 1.60 - 1.37 (m, 4H), 1.26 (s, 6H).

ESI - LRMS : 260.2 [M+H]+

**Preparation of S-methyl 4-((4-(benzyloxy)butyl)(methyl)amino)-4-methylpent-2-ynethioate** : The compound was obtained by using the same process as the one described for S-methyl 4-[2-ethoxyethyl(methyl)amino]-4-methyl-pent-2-ynethioate [example 1] starting from N-(4-benzyloxybutyl)-N,2-dimethyl-but-3-yn-2-amine . Purification by chromatography on silicagel (cyclohexane/EtOAc= 90/10). Scale 0.77 mmol. Yield : 80%. Yellow oil.

1H NMR (300MHz, CDCl3) δ 7.38 - 7.19 (m, 5H), 4.48 (s, 2H), 3.47 (t, J = 6.1 Hz, 2H), 2.45 (t, J = 6.9Hz, 2H), 2.35 (s, 3H), 2.26 (s, 3H), 1.73 - 1.48 (m, 4H), 1.39 (s, 6H).

ESI - HRMS : calc for C19H28NO2S [M+H]+ 334.1835, found 334.1840.

**Example 18: S-methyl 4-((2-hydroxyethyl)(methyl)amino)-4-methylpent-2-ynethioate**

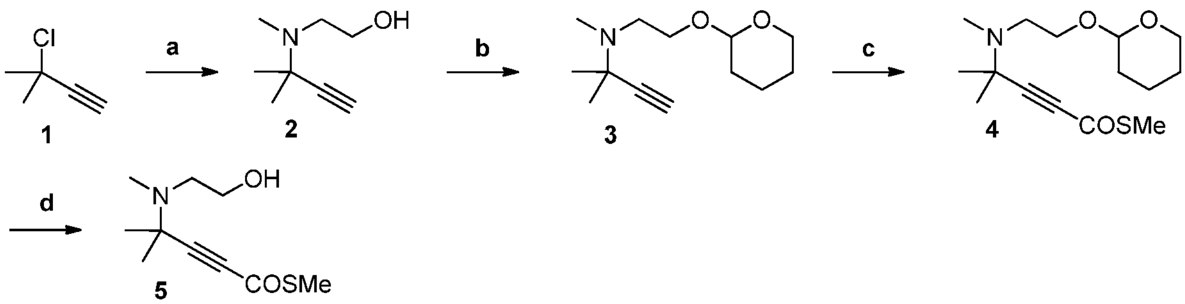


1 2 3 4 5

**a)** see Easton, Nelson R.; Hennion, George F. , U.S. (1966), US 3285913 b) 3,4-DHP, pTSA, DCM (70%) c) nBuLi, THF, -70°C, carbonyl sulfide then MeI 0°C (59%) d) pTSA, MeOH, room temperature (90%)

**Preparation of Compound 3: N,2-dimethyl-N-(2-((tetrahydro-2H-pyran-2-yl)oxy)ethyl)but-3-yn-2-amine:** To 2-(methyl(2-methylbut-3-yn-2-yl)amino)ethanol 2 (3.00 g, 21.2 mmol) and 3,4-Dihydro-2H-pyran (5.0 eq) in anhydrous DCM (135 mL) was added p-toluenesulfonic acid (0.1 eq) at room temperature. The reaction mixture was stirred overnight, washed with aqueous saturated $NaHCO_3$ (30 mL) then brine (30 mL). The organic layer was dried over sodium sulfate, filtered and concentrated *in vacuo.* The residue was first purified by short-path distillation using Kugelrohr apparatus (10-12 Torrs, oven 155°C) then by flash chromatography on silica gel (petroleum ether/ethyl acetate 95/5 to 60/40) to give compound 3 as an oil (yield 70%).

$^1$H NMR (300 MHz, DMSO) δ 4.60 - 4.50 (m, 1H), 3.82-3.70 (m, 1H), 3.59-3.56 (m, 1H), 3.49 - 3.35 (m, 2H), 3.12 (s, 1H), 2.55 (t, *J* = 6.5 Hz, 2H), 2.21 (s, 3H), 1.77 - 1.35 (m, 6H), 1.27 (s, 6H).

**Preparation of Compound 4: S-methyl 4-methyl-4-(methyl(2-((tetrahydro-2H-pyran-2-yl)oxy) ethyl) amino) pent-2-ynethioate:** To the acetylenic amine **3** (1.00 g, 4.44 mmol) in anhydrous THF (22 mL) was added n-Butyllithium solution (2.2 M in hexanes, 1.5 eq) dropwise. The mixture was allowed to reach to 0°C within 10 minutes then re-cooled

to - 70°C before carbonyl sulfide bubbling. After 30 minutes the bright yellow solution was carefully warmed to 0°C, stirred 30 minutes at this temperature and methyl iodide (1.2 eq) was added dropwise. The reaction mixture was stirred for 2 hours at 0°C before hydrolysis by water. Extractive work-up by DCM (washing with brine, drying with sodium sulfate and concentration under reduced pressure) gave a crude which was purified by chromatography on silica gel (petroleum ether/ethyl acetate 90/10 to 60/40) to give compound **4** as an oil (yield 59%).

$^{1}H$ NMR (300 MHz, DMSO) δ 4.58 (t, *J* = 3.2 Hz, 1H), 3.75 (ddd, J= 11.4, 7.9, 3.3 Hz, 1H), 3.70 - 3.60 (m, 1H), 3.49 - 3.38 (m, 2H), 2.59 (t, *J* = 6.3 Hz, 2H), 2.39 (s, 3H), 2.27 (s, 3H), 1.77 - 1.39 (m, 6H), 1.36 (s, 6H). $^{13}C$ NMR (75 MHz, $CDCl_3$) δ 176.62 (C=O), 98.94 (CH), 96.46 (C), 80.97 (C), 66.55 ($CH_2$), 62.37 ($CH_2$), 55.19 (C), 52.43 ($CH_2$), 37.92 ($CH_3$), 30.75 ($CH_2$), 28.01(2×$CH_3$), 25.59 ($CH_2$), 19.63 ($CH_2$), 12.61 ($CH_3$).

**Preparation of Compound 5: S-methyl 4-((2-hydroxyethyl)(methyl)amino)-4-methylpent-2-ynethioate:** To the aminothiolester **4** (1.00 g, 3.34 mmol) in methanol (15 mL) was added p-toluenesulfonic acid (1.1 eq) at room temperature. The reaction mixture was stirred overnight, washed with aqueous saturated $NaHCO_3$ (30 mL) then brine (30 mL). The organic layer was dried over sodium sulfate, filtered and concentrated *in vacuo.* The residue was purified by flash chromatography on silica gel (petroleum ether/ethyl acetate 80/20 to 20/80) to give compound 5 as an oil (yield 90%).

$^{1}H$ NMR (300 MHz, DMSO) δ 4.42 (t, *J* = 5.6 Hz, 1H), 3.44 (td, *J* = 6.7, 5.6 Hz, 2H), 2.46 (, *J* = 6.7 Hz, 2H), 2.39 (s, 3H), 2.24 (s, 3H), 1.36 (s, 6H). $^{13}C$ NMR (75 MHz, $CDCl_3$) δ 176.46 (C=O), 95.56 (C), 80.89 (C), 58.92 ($CH_2$), 54.99 (C) , 53.52 ($CH_2$), 36.12 ($CH_3$), 27.88 (2×$CH_3$), 12.53($CH_3$). ESI-HRMS: Calc. for $C_{10}H_{18}NO_2S$ $[M+H]^+$ 216.1053 found 216.1043.

## Example 19. S-methyl 4-methyl-4-[methyl-[2-(2-naphthylmethoxy)ethyl]amino]pent-2-ynethioate

**Preparation of N,2-dimethyl-N-[2-(2-naphthylmethoxy)ethyl]but-3-yn-2-amine:** The compound is obtained by using the same process as the one described for N-(2-allyloxyethyl)-N,2-dimethyl-but-3-yn-2-amine [example 2] using 1.015 eq of NaH and 1.01 eq. of 2-naphtyl bromide. Purification by chromatography on silicagel (petroleum ether/EtOAc=90/10). Yield: 61%. orange oil.

1H NMR (300 MHz, DMSO) δ 7.91 - 7.88 (m, 4H), 7.50 - 7.48 (m, 3H), 4.65 (s, 2H), 3.55 (t, 6.1Hz, 2H), 3.13 (s, 1H), 2.62 (t, 6.2Hz, 2H), 2.23 (s, 3H), 1.28 (s, 6H).

**Preparation of S-methyl 4-methyl-4-[methyl-[2-(2-naphthylmethoxy)ethyl]amino]pent-2-ynethioate:** The compound is obtained by using the same process as the one described for S-methyl 4-[2-ethoxyethyl(methyl)amino]-4-methyl-pent-2-ynethioate [example 1] starting from N,2-dimethyl-N-[2-(2-naphthylmethoxy)ethyl]but-3-yn-2-amine. Scale: 0.65 mmol. Purification by chromatography on silicagel (petroleum ether/EtOAc=85/15). Yield: 28%. Yellow oil.

1H NMR (300 MHz, DMSO) δ7.95 - 7.82 (m, 4H), 7.55 - 7.41 (m, 3H), 4.66 (s, 2H), 3.57 (t, J = 6.2Hz, 2H), 2.66 (t, J = 6.1 Hz, 2H), 2.38 (s, 3H), 2.28 (s, 3H), 1.37 (s, 6H).

ESI - HRMS : calc. for $C_{21}H_{26}NO_2S$ 356.1683, found 356.1679 [M+H]+

## Example 20: S-methyl 4-methyl-4-[methyl-[2-[(2,6,6-trimethylcyclohexen-1-yl)methoxy]ethyl]amino]pent-2-ynethioate

**Preparation of N,2-dimethyl-N-[2-[(2,6,6-trimethylcyclohexen-1-yl)methoxy]ethyl]but-3-yn-2-amine:** The compound is obtained by using the same process as the one described for N-(2-allyloxyethyl)-N,2-dimethyl-but-3-yn-2-amine [example 2] using 1.015 eq of NaH and 1.01 eq. of 2-(Bromomethyl)-1,3,3-trimethyl-1-cyclohexene (prepared from β-Cyclocitral by known protocols (WO 2015048363)). Purification by chromatography on silicagel (petroleum ether/EtOAc=95/05). Yield: 67%. Pale yellow oil.

3.86 (s, 2H), 3.41 (t, J = 6.5Hz, 2H), 3.12 (s, 1H), 2.53 (t, J = 6.4Hz, 2H), 2.20 (s, 3H), 1.90 (t, J = 5.9Hz, 2H), 1.62 (s, 3H), 1.57 - 1.49 (m, 2H), 1.41 - 1.33 (s, 2H), 1.27 (s, 6H), 0.97 (s, 6H).

**Preparation of S S-methyl 4-methyl-4-[methyl-[2-[(2,6,6-trimethylcyclohexen-1-yl)methoxy]ethyl]amino]pent-2-ynethioate:** The compound is obtained by using the same process as the one described for S-methyl 4-[2-ethoxyethyl(methyl)amino]-4-methyl-pent-2-ynethioate (example 19) starting from N,2-dimethyl-N-[2-[(2,6,6-trimethylcyclohexen-1-yl)methoxy]ethyl]but-3-yn-2-amine. Purification by chromatography on silicagel (petroleum ether/EtOAc=90/10). Yield: 63%. Yellow oil.

H NMR (300 MHz, DMSO) δ 3.87 (s, 2H), 3.44 (t, J = 6.3 Hz, 2H), 2.57 (t, J = 6.3 Hz, 2H), 2.38 (s, 3H), 2.26 (s, 3H), 1.91 (t, J = 6.2 Hz, 2H), 1.62 (s, 3H), 1.57 - 1.49 (m, 2H), 1.36 (s, 6H), 1.39 - 1.34 (m, 2H), 0.97 (s, 6H).

ESI - HRMS calc for C20H34NO2S [M+H]+: 352.2305, found : 352.2289.

**Example 21: 2-[(1,1-dimethyl-4-methylsulfanyl-4-oxo-but-2-ynyl)-methylamino]ethyl acetate:**

To S-methyl 4-[2-hydroxyethyl(methyl)amino]-4-methyl-pent-2-ynethioate (100 mg, 0.46 mmol) and and di-isopropylethylamine (1.2eq) in dichloromethane (2.3 mL) was added dropwise acetyl chloride (1.2eq) at 0°C. After 10 min at 0°C the reaction mixture was warmed up to rt and stirred until complete conversion (TLC checking). The mixture

is then diluted in dichloromethane (30 mL), washed with brine (40 mL), dried over sodium sulfate and the solvent evaporated under reduced pressure. The crude was purified by chromatography on silica gel (petroleum ether/ethyl acetate 75/25) to give product 21 as a yellow oil. Yield 58%.

1H NMR (300MHz, DMSO) δ 4.05 (t, J = 6.1Hz, 2H), 2.62 (t, J = 6.1Hz, 2H), 2.39 (s, 3H), 2.26 (s, 3H), 2.01 (s,3H), 1.36 (s, 6H).

ESI -HRMS calc for C12H19NO3S [M+H]+: 258.1086, found : 258.1150.

**Example 22: 2-[(1,1-dimethyl-4-methylsulfanyl-4-oxo-but-2-ynyl)-methylamino]ethyl-3,4-dimethoxybenzoate:**

The compound is obtained by using the same process as the one described for 2-[(1,1-dimethyl-4-methylsulfanyl-4-oxo-but-2-ynyl)-methylamino]ethyl acetate [example 21] starting from 3,4-dimethoxybenzoyl chloride. Scale: 0.46 mmol. Purification by chromatography on silica gel (petroleum ether/EtOAc=60/40). Yield: <10%. yellow oil.

1H NMR (300 MHz, DMSO) δ 7.59 (dd, J = 8.4, 2.0Hz, 1H), 7.45 (d, J = 2.0Hz, 1H), 7.08 (d,J = 8.5Hz, 1H), 4.28 (t, J = 7.1Hz, 2H), 3.83 (s, 3H), 3.80 (s, 3H), 2.72 (t, J = 7.1Hz, 2H), 2.38(s, 3H), 2.33 (s, 3H), 1.39 (s, 6H).

**Example 23: S-methyl 2,5,10,11,11-pentamethyl-6-oxo-7-oxa-2,5,10-triazatetradec-12-yne-14-thioate**

The compound is obtained by using the same process as the one described for S-methyl 4-methyl-4-[methyl-[2-[methyl-[2-(methylamino)ethyl]carbamoyl]oxyethyl]amino]pent-2-ynethioate [compound 7 / Example 29] starting from N,N,N'-Trimethylethylenediamine. Purification by chromatography on silica gel DCM/MeOH (85/15). Yield: 34%. Yellow oil. 1H NMR (300 MHz, DMSO) δ 4.01 (t, J = 5.9Hz, 2H), 3.32-3.25 (m, 2H), 2.83 (large s, 3H), 2.62 (t, J = 5.8Hz, 2H), 2.6-2.5 (m partially hide by solvent peak, 2H), 2.39 (s, 3H), 2.27 (s,3H), 2.17 (large s, 6H), 1.36 (s, 6H).

ESI-HRMS calc for C16H30N3O3S [M+H]+: 344.1992, found : 344.1993.

**Example 24: (S)-S-methyl 4-(2-(methoxymethyl)pyrrolidin-1-yl)-4-methylpent-2-ynethioate**

**Preparation of (S)-2-(methoxymethyl)-1-(2-methylbut-3-yn-2-yl)pyrrolidine** : The compound was obtained by using the same process as the one described for N-(2-allyloxyethyl)-N,2-dimethyl-but-3-yn-2-amine [example 2] using 1eq of (S)-pyrrolidin-2-ylmethanol, 1.015 eq of NaH and 1.01 eq. of iodomethane. Purification by chromatography on silicagel (dichloromethane/methanol = 90/10). Scale 3.0 mmol. Yield : 61%. Orange oil. $^1$H NMR (300 MHz, DMSO) δ 3.23 (s, 3H), 3.16 - 3.06 (m, 2H), 3.05 (s, 1H), 3.03 - 2.91 (m, 1H), 2.91 - 2.80 (m, 1H), 2.67 - 2.54 (m, 1H), 1.77 - 1.49 (m, 4H), 1.30 (s, 3H), 1.24 (s, 3H).

ESI - LRMS : 182.2 [M+H]+.

**Preparation of (S)-S-methyl 4-(2-(methoxymethyl)pyrrolidin-1-yl)-4-methylpent-2-ynethioate** : The compound was obtained by using the same process as the one described for S-methyl 4-[2-ethoxyethyl(methyl)amino]-4-methyl-pent-2-ynethioate [example 1] starting from (S)-2-(methoxymethyl)-1-(2-methylbut-3-yn-2-yl)pyrrolidine. Purification by chromatography on silicagel (petroleum ether/EtOAc= 80/20). Scale 0.8 mmol. Yield : 57%. Yellow oil.

$^1$H NMR (300 MHz, DMSO) δ 3.24 (s, 3H), 3.19 - 2.89 (m, 4H), 2.67 - 2.53 (m, 1H), 2.38 (s, 3H), 1.83 - 1.50 (m, 4H), 1.39 (s, 3H), 1.33 (s, 3H).

ESI- HRMS calc for $C_{13}H_{22}NO_2S$ [M+H]+: 256.1366, found: 256.1363.

**Example 25: S-methyl 4-[(3R)-3-methoxypyrrolidin-1-yl]-4-methyl-pent-2-ynethioate**

**Preparation of (R)-3-methoxy-1-(2-methylbut-3-yn-2-yl)pyrrolidine** : The compound was obtained by using the same process as the one described for N-(2-allyloxyethyl)-N,2-dimethyl-but-3-yn-2-amine [example 2] starting from (R)-1-(2-methylbut-3-yn-2-yl)pyrrolidin-3-ol using 1.015 eq of NaH and 1.01 eq. of iodomethane. Purification by chromatography on silicagel (dichloromethane/methanol = 90/10). Scale 3.3 mmol. Yield : 30%. Yellow oil.

$^1$H NMR (300 MHz, DMSO) δ 3.85 (ddd, *J* = 10.8, 7.2, 3.6 Hz, 1H), 3.17 (s, 3H), 3.13 (s, 1H) 2.82 (dd, *J* = 9.8, 6.5 Hz, 1H), 2.66 - 2.60 (m, 1H), 2.56 - 2.50 (m, 2H), 2.01 - 1.87 (m, 1H), 1.73 - 1.55 (m, 1H), 1.28 (s, 6H).

ESI - LRMS: 168.0 $[M+H]^+$.

**Preparation of (R)-S-methyl 4-(3-methoxypyrrolidin-1-yl)-4-methylpent-2-ynethioate** : The compound was obtained by using the same process as the one described for S-methyl 4-[2-ethoxyethyl(methyl)amino]-4-methyl-pent-2-ynethioate [example 1] starting from (R)-3-methoxy-1-(2-methylbut-3-yn-2-yl)pyrrolidine. Purification by chromatography on silicagel (petroleum ether/EtOAc=60/40). Scale 0.8 mmol. Yield : 55%. Yellow oil.

$^1$H NMR (300 MHz, DMSO) δ 3.87 (ddd, J = 10.5, 6.9, 3.4 Hz, 1H), 3.18 (s, 3H), 2.90 - 2.76 (m, 1H), 2.77 - 2.65 (m, 1H), 2.65 - 2.54 (m, 2H), 2.38 (s, 3H), 2.07 - 1.86 (m, 1H), 1.76 - 1.60 (m, 1H), 1.36 (s, 6H).

**Example 26: S-methyl 4-(((1R,2R)-2-(benzyloxy)cyclopentyl)(methyl)amino)-4-methylpent-2-ynethioate**

**Preparation of (1R, 2R)-2-benzyloxy-N-(1,1-dimethylprop-2-ynyl)cyclopentanamine** : To a solution of commercially available (1R,2R)-2-(benzyloxy)cyclopentanamine (0.93 g, 4.86 mmol), 3-chloro-3-methylbut-1-yne (1.3eq) and triethylamine (1.3eq) in THF (20 mL) was added CuI (8 mol%) at room temperature. The mixture was left to stir overnight. The solvent was evaporated under reduced pressure and the crude was then diluted in aqueous saturated $NaHCO_3$ solution, extracted with ethyl acetate. Combined organic layers were washed with 2% $NH_4OH$ aqueous solution then brine, dried over $Na_2SO_4$ and the solvent evaporated under reduced pressure. (1R, 2R)-2-benzyloxy-N-(1,1-dimethylprop-2-ynyl)cyclopentanamine was obtained as a brown oil. Yield : 99%.

$^1H$ NMR (300 MHz, DMSO) $\delta$ 7.37 - 7.16 (m, 5H), 4.56-4.36 (m, 2H), 3.72 -3.58 (m, 1H), 3.28 - 3.19 (m, 1H), 3.08 (s, 1H), 2.00 - 1.88 (m, 1H), 1.86- 1.67 (m, 2H), 1.65- 1.49 (m, 3H), 1.40 - 1.28 (m, 1H), 1.25 (s, 3H), 1.25 (s, 3H).

**Preparation of (1R,2R)-2-(benzyloxy)-N-methyl-N-(2-methylbut-3-yn-2-yl)cyclopentanamine** : To (1R,2R)-2-(benzyloxy)-N-(2-methylbut-3-yn-2-yl)cyclopentanamine (0.25 g, 0.97 mmol) were added 5 eq of formic acid and 1.5 eq of formaldehyde (37% in water). The mixture was refluxed overnight then 2N HCI was added until pH 1 was reached and washed with ether. The aqueous layer was basified with 1N NaOH, and extracted with DCM. The organic layer was washed with brine, dried over $Na_2SO_4$ and the solvents evaporated under reduced pressure. The crude is then purified by chromatography on silica gel (petroleum ether/EtOAc = 80/20), giving a yellow oil. Yield : 68%.

$^1H$ NMR (300 MHz, DMSO) $\delta$ 7.43 - 7.17 (m, 5H), 4.52(m, 2H), 3.94 - 3.78 (m, 1H), 3.62 - 3.49 (m, 1H), 3.11 (s, 1H), 2.20 (s, 3H), 1.78 - 1.45 (m, 6H), 1.37 (s, 3H), 1.34 (s, 3H). ESI - LRMS: 272.1 $[M+H]^+$.

**Preparation of S-methyl 4-(((1R,2R)-2-(benzyloxy)cyclopentyl)(methyl)amino)-4-methylpent-2-ynethioate** : The compound was obtained by using the same process as the one described for S-methyl 4-[2-ethoxyethyl(methyl)amino]-4-methyl-pent-2-ynethioate [example 1] starting from (1R,2R)-2-(benzyloxy)-N-methyl-N-(2-methylbut-3-yn-2-yl)cyclopentanamine. Purification by chromatography on silicagel (petroleum ether/EtOAc= 80/20 then DCM = 100). Scale 0.64 mmol. Yield : 47%. Yellow oil.

$^1H$ NMR (300 MHz, DMSO) $\delta$ 7.35 - 7.20 (m, 5H), 4.51 (s, 2H), 3.92 - 3.80 (m, 1H), 3.59 - 3.45 (m, 1H), 2.36 (s, 3H), 2.24 (s, 3H), 1.78 - 1.49 (m, 6H), 1.45 (s, 3H), 1.42 (s, 3H). ESI - HRMS calc for $C_{29}H_{23}NO_2S$ [M+H]+: 346.1835, found: 346.1824.

**Example 27: (S)-S-methyl 4-(2-((benzyloxy)methyl)pyrrolidin-1-yl)-4-methylpent-2-ynethioate:**

**Preparation of (S)-2-((benzyloxy)methyl)-1-(2-methylbut-3-yn-2-yl)pyrrolidine:** The compound was obtained by using the same process as the one described for N-(2-allyloxyethyl)-N,2-dimethyl-but-3-yn-2-amine [example 2] starting from (S)-(1-(2-methylbut-3-yn-2-yl)pyrrolidin-2-yl)methanol 1.015 eq of NaH and 1.01 eq. of benzyl bromide. Purification by chromatography on silicagel (dichloromethane/methanol = 95/5). Scale 3.0 mmol. Yield : 60%. Orange oil.

$^1H$ NMR (300 MHz, DMSO) $\delta$ 7.41 - 7.19 (m, 5H), 4.46 (s, 2H), 3.24 (dd, J = 8.3, 2.7 Hz, 1H), 3.14 (dd, J = 7.6, 3.2 Hz, 1H), 3.11 - 3.02 (m, 2H), 2.86 (dd, J = 8.4, 6.0 Hz, 1H), 2.65 - 2.54 (m, 1H), 1.81 - 1.51 (m, 4H), 1.25 (s, 3H), 1.23 (s, 3H).

ESI-LRMS: 258.1 [M+H]+.

**Preparation of (S)-S-methyl 4-(2-((benzyloxy)methyl)pyrrolidin-1-yl)-4-methylpent-2-ynethioate:** The compound was obtained by using the same process as the one described for S-methyl 4-[2-ethoxyethyl(methyl)amino]-4-methyl-pent-2-ynethioate [example 1] starting from (S)-2-((benzyloxy)methyl)-1-(2-methylbut-3-yn-2-yl)pyrrolidine. Purification by chromatography on silicagel (petroleum ether/EtOAc=80/20). Scale 1.2 mmol. Yield : 50%. Orange oil.

$^1H$ NMR (300 MHz, DMSO) $\delta$ 7.46 - 7.18 (m, 5H), 4.47 (s, 2H), 3.29 - 3.23 (m, 1H), 3.17 - 3.03 (m, 2H), 2.94 (dd, J = 8.5, 5.7 Hz, 1H), 2.62 - 2.53 (m, 1H), 2.38 (s, 3H), 1.85 - 1.58 (m, 4H), 1.34 (s, 3H), 1.32 (s, 3H).

**Example 28: (R)-S-methyl 4-(3-(benzyloxy)pyrrolidin-1-yl)-4-methylpent-2-ynethioate:**

**Preparation of (R)-3-(benzyloxy)-1-(2-methylbut-3-yn-2-yl)pyrrolidine** : The compound was obtained by using the same process as the one described for N-(2-allyloxyethyl)-N,2-dimethyl-but-3-yn-2-amine [example 2] starting from (R)-1-(2-methylbut-3-yn-2-yl)pyrrolidin-3-ol using 1.01 eq of NaH and 1.01 eq. of benzyl bromide. Purification by chromatography on silicagel (dichloromethane/methanol = 98/2). Scale 3.3 mmol. Yield : 69%. Orange oil.

$^1H$ NMR (300 MHz, DMSO) $\delta$ 7.40 - 7.17 (m, 5H), 4.42 (s, 2H), 4.07 (tt, J = 7.3, 3.7Hz, 1H) 3.13 (s, 1H), 2.86 (dd, J = 9.6, 6.7 Hz, 1H), 2.75 - 2.65 (m, 2H), 2.60 - 2.53 (m, 1H), 2.06 - 1.95 (m, 1H), 1.75 - 1.65 (m, 1H), 1.29 (s, 6H).

ESI - LRMS 244.1 [M+H]+.

**Preparation of (R)-S-methyl 4-(3-(benzyloxy)pyrrolidin-1-yl)-4-methylpent-2-ynethioate:** The compound was obtained by using the same process as the one described for S-methyl 4-[2-ethoxyethyl(methyl)amino]-4-methyl-pent-2-ynethioate [example 1] starting from (R)-3-(benzyloxy)-1-(2-methylbut-3-yn-2-yl)pyrrolidine. Purification by chromatography on silicagel (dichloromethane/methanol=98/2). Scale 1.2 mmol. Yield: 69%. Orange oil.

$^1H$ NMR (300 MHz, DMSO) δ 7.32 (m, 5H), 4.44 (s, 2H), 4.09 (dq, J = 10.2, 3.5 Hz, 1H), 2.97 - 2.83 (m, 1H), 2.80 - 2.65 (m, 2H), 2.65 - 2.55 (m, 1H), 2.37 (s, 3H), 2.11 - 1.90 (m, 1H), 1.83 - 1.67 (m, 1H), 1.37 (s, 6H).
ESI - HRMS calc for $C_{18}H_{24}NO_2S$ [M+H]+: 318.1522, found: 318.1518.

**Example 29: ADC compound (without antibody)**

**Preparation of a linker (Mc-Val-Cit-PAB-PNP):** The selected linker for the conjugate preparation was designed on known platform already used in Rituximab and others, comprising the maleimide for attachment to the Antibody, the Cathepsin cleavable group and the p-amino benzyl system for the 1,6-elimination: Mc-Val-Cit-PAB-PNP, CAS 159857-81-5.
It was prepared following standard protocols starting from Fmoc-Val-OSu [CAS 3392-12-9] or may be purchased from commercial suppliers (ex. creative biolabs, ALB technology, Carbosynth etc.).
The general formula of the linker is indicated below:

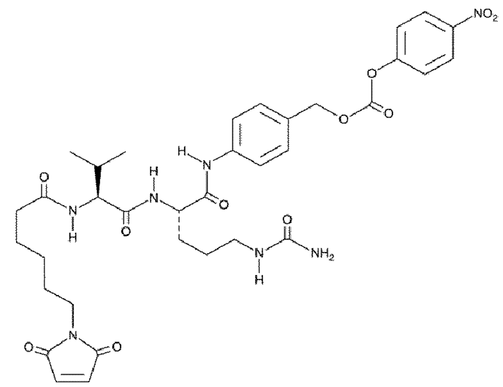

Preparation of the compound according to the invention (example 18) coupled to Mc-Val-Cit-PAB-PNP

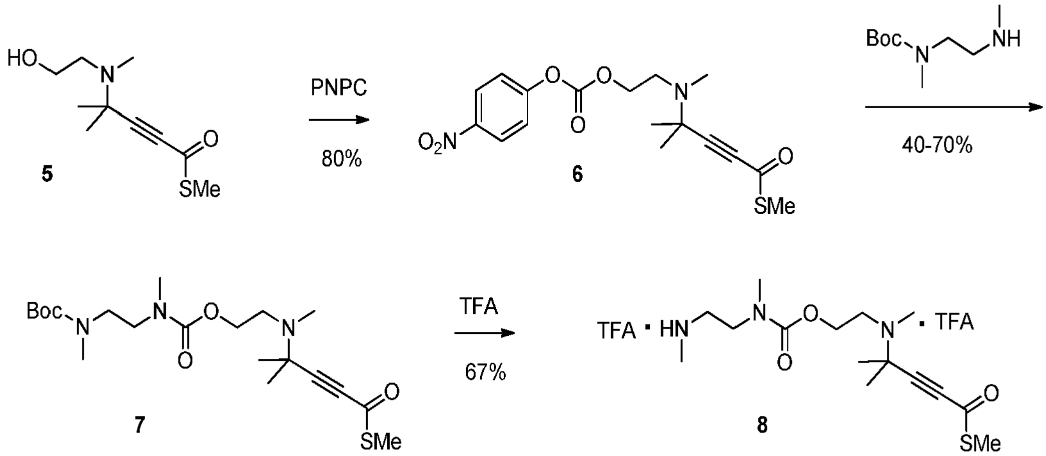


5

6

7

8

**Compound 6:**

To p-nitrochloroformate (140 mg, 0.70mmol, 1.5eq) in of DCM (2.5 mL) was added dropwise a solution of compound 5 (100mg, 0.46mmol) and TEA (1.5eq) in DCM (1.5 mL) at 0°C. After 10 minutes at 0°C the reaction mixture was warmed up to room temperature and stirred until complete conversion (TLC checking, 1h). The mixture is then diluted in DCM (30 mL), washed with brine (40 mL), dried over $Na_2SO_4$ and the solvent evaporated under reduced pressure. The crude was purified by chromatography on silica gel (petroleum ether/ethyl acetate 100/ to 70/30) to give compound 6 as an amorphous solid (yield 82%). $^1H$ NMR (300MHz, DMSO): δ(ppm) 8.35-8.30 (m, 2H), 7.59-7.53 (m, 2H), 4.30 (t, $J$ = 5.7 Hz, 2H), 2.75 (t, J= 5.7 Hz, 2H), 2.39 (s, 3H), 2.30 (s, 3H), 1.39 (s, 6H).

**Compound 7:**

To compound 6 (200mg, 0.53mmol) in DCM (3 mL) is added TEA (1.2eq) at room temperature. Then a solution of tert-butyl N-methyl-N-[2-(methylamino)ethyl]carbamate (1.2eq) in DCM (2.3 mL) is added at 0°C. The bright yellow reaction mixture obtained was warmed up to room temperature and stirred overnight. The mixture was diluted in 30mL of DCM, washed by 40 mL of brine, dried over $Na_2SO_4$ and the solvent evaporated under reduced pressure. The crude was purified by chromatography on silica gel (petroleum ether/ethyl acetate 75/25 to 20/80) to give compound 7 as a visquous oil (yield 70%).

$^1$H NMR (300MHz, DMSO) : δ(ppm) 3.99 (t, *J* = 5.7Hz, 2H), 3.30 (s, 3H), 2.85-2.70 (m, 7H), 2.66-2.58 (m, 2H), 2.38 (s, 3H), 2.26 (s, 3H), 1.37 (s, 9H), 1.36 (s, 6H). ESI - LRMS : Calc. for $C_{20}H_{36}N_3O_5S$ $[M+H]^+$ : 430.2 ; found : 430.2.

**Compound 8:**

To compound 7 (44.5mg, 0.104mmol) in 4.5mL of DCM (4.5 mL) was added TFA (0.5 mL) at 0°C. The mixture was then allowed to warm up to room temperature and stirred overnight. After concentration *in vacuo* (bath T°C < 45°C) The oily residue was triturated and sonicated in $Et_2O$. The resulting sticky solid (bis trifluoroacetate salt) was washed with $Et_2O$ and dried. Yield : 67%.

$^1$H NMR (300 MHz, $D_2O$) δ 4.57 - 4.44 (m, 2H), 3.73 - 3.69 (m, 2H), 3.62 (t, *J* = 5.7 Hz, 2H), 3.30 - 3.19 (t, *J* = 5.8 Hz, 2H), 3.05 - 2.99 (m, 3H), 2.99 - 2.90 (m, 3H), 2.73 (s, 3H), 2.45 (s, 3H), 1.79 (s, 6H). ESI - LRMS : Calc. for $C_{15}H_{28}N_3O_3S$ $[M+H]^+$ : 330.2 ; found : 330.1.

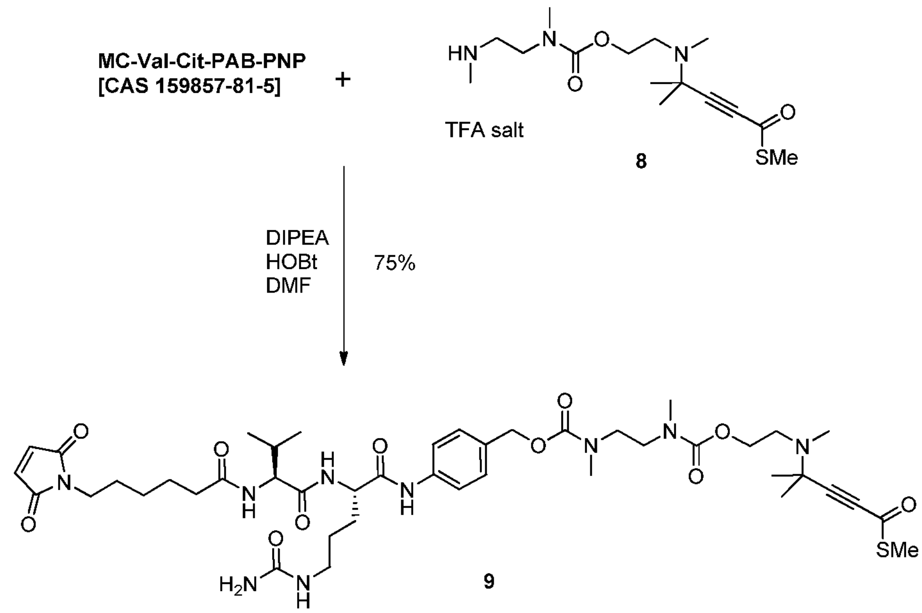


**Compound 9:**

To MC-Val-Cit-PABC-PNP (52mg, 0.07mmol) and compound **8** (1.06eq) in DMF (1.4 mL) were sequentially added at 0°C : HOBt (1eq) in one portion then DIPEA (3eq) dropwise. After 5 minutes at 0°C, the mixture was warmed up to room temperature and stirred overnight. The mixture is then concentrated under vacuum (¾ evaporation off, bath T°C<45°C) and flocculated with $Et_2O$. A white crude solid was obtained after washings/triturations (x3) in $Et_2O$. A final purification by chromatography on silica (MeOH/DCM 95/5 to 90/10%) gave the final product (compound 9) as a white solid. Yield : 75%.

$^1$H NMR (300 MHz, DMSO) δ 9.99 (s, 1H), 8.08 (d, *J* = 7.4 Hz, 1H), 7.80 (d, *J* = 8.6 Hz, 1H), 7.59 (d, *J* = 8.5 Hz, 2H), 7.35 - 7.22 (m, 2H), 7.01 (s, 2H), 5.97 (t, *J* = 5.8 Hz, 1H), 5.41 (s, 2H), 4.98 (s, 2H), 4.44 - 4.32 (m, 1 H), 4.25 - 4.14 (m, 1H), 3.99 (s, 2H), 3.45 - 3.33 (m, 4H), 3.09 - 2.89 (m, 2H), 2.89 - 2.70 (m, 6H), 2.65 - 2.56 (m, 2H), 2.38 (s, 3H), 2.30 - 2.11 (m, 5H), 2.10-1.90 (m, 1H), 1.77 - 1.09 (m, 18H), 0.84 (dd, *J* = 9.5, 6.8 Hz, 6H).

LC : Zorbax, ACN/$H_2O$ 0.1% TFA, 254 nm, 96%.

ESI-HRMS : Calc. for $C_{44}H_{66}N_9O_{11}S$ $[M+H]^+$ 928.4597 found 928.4586.

## Part 2: Use of a compound according to the invention

### Example 1: activity of the compounds according to the invention *Material and Methods*

**Cell lines.** Leukemic cell line, HL-60 (derived from a 36-year-old female with AML-M2), was used for determination of drug efficacy. Cells were obtained from the European Collection of Cell Cultures (ECACC). All cells were cultivated in appropriate media according to supplier recommendations.

**Cell Viability assay, 96-well format.** Cells were seeded into 96-well cell culture plates at concentrations required to ensure approximately 80% confluence in control (untreated cells) at the end of experiment ($0.5 \times 10^4$ - $5 \times 10^4$ cell/well).

The sensitivity towards compounds according to the invention was determined using different concentrations of each compound ranging from 0.5 to 100 μM (0.5, 1, 2, 5, 10, 15, 20, 25, 30, 40, 50, 100 μM). Following 48 hours of incubation at 37°C in a humidified atmosphere containing 5% $CO_2$, the growth-inhibitory effect of compounds was analyzed using Resazurin, according to manufactures instructions.

To ensure good data quality and to minimize impact of pipetting errors, each compounds concentration was assessed based on mean fluorescence intensity from 8 separate wells. Compounds response were quantified by the half maximal inhibitory concentration ($IC_{50}$) for each particular cell line, and determined by non-linear regression analysis of log-dose/response curves.

**Statistical Analysis.** Values were expressed as mean ± SD or frequencies and proportions. Cell viability curves were determined using four parameter regression line. Differences between groups were determined by unpaired t test, Chi-square, Fisher's exact test or ANOVA, where appropriate. $P < 0.05$ was considered statistically significant. Analysis was performed using GraphPad prism version 5.0 (GraphPad software, San Diego California USA).

### *Results*

The $IC_{50}$ obtained by testing the cytotoxicity activity of the compounds according to the invention (except compounds 13, 14, 15 and 16 which are not part of the invention) in HL60 cells are mentioned in table 3 below.

**Table 3**

| Example | Name | $IC_{50}$ at 48h |
|---|---|---|
| 1 | S-methyl 4-[2-ethoxyethyl(methyl)amino]-4-methyl-pent-2-yneth ioate | 4.977 μM |
| 2 | S-methyl 4-[2-allyloxyethyl(methyl)amino]-4-methyl-pent-2-yneth ioate | 6.5 μM |
| 3 | S-methyl 4-[2-benzyloxyethyl(methyl)amino]-4-methyl-pent-2-yneth ioate | 1.169 μM |
| 4 | S-methyl 4-methyl-4-[methyl-[2-(m-tolylmethoxy)ethyl]amino]pent-2-ynethioate | 5.622 μM |
| 5 | S-methyl 4-[2-[(3,4-dimethylphenyl)methoxy]ethylmethyl-amino]-4-methyl-pent -2-ynethioate | 4.722 μM |
| 6 | S-methyl 4-[2-[(4-methoxyphenyl)methoxy]ethylmethyl-amino]-4-methyl-pent -2-ynethioate | 6.478 μM |
| 7 | S-methyl 4-[2-[(3,4-dimethoxyphenyl)methoxy]ethylmethyl-amino]-4-methyl-pent -2-ynethioate | 6.085 μM |
| 8 | S-methyl 4-[2-[(3-chlorophenyl)methoxy]ethyl-methyl-amino]-4-methyl-pent-2-ynethioate | 7.069 μM |
| 9 | S-methyl 4-[2-[(3-fluorophenyl)methoxy]ethyl-methyl-amino]-4-methyl-pent-2-ynethioate | 6.647 μM |
| 10 | S-methyl 4-methyl-4-[methyl-[2-(2-pyridylmethoxy)ethyl]amino]pent-2-ynethioate | 5.622 μM |
| 11 | S-methyl 4-methyl-4-[methyl-[2-(3-pyridylmethoxy)ethyl]amino]pent-2-ynethioate | 6.560 μM |
| 12 | S-methyl 4-methyl-4-[methyl-[2-(4-pyridylmethoxy)ethyl]amino]pent-2-ynethioate | 5.684 μM |

(continued)

| Example | Name | $IC_{50}$ at 48h |
|---|---|---|
| 13 | methyl 4-(dimethylamino)-4-methyl-pent-2-ynoate | 42.66 μM (is not part of the invention) |
| 14 | ethyl 4-(dimethylamino)-4-methyl-pent-2-ynoate | 40.00 μM (is not part of the invention) |
| 15 | tert-butyl 2-((4-(dimethylamino)-4-methylpent-2-ynoyl)thio)acetate | 25 μM (is not part of the invention) |
| 16 | 2-((4-(dimethylamino)-4-methylpent-2-ynoyl)thio)acetic acid | 22.48 μM (is not part of the invention) |
| 17 | S-methyl 4-((4-(benzyloxy)butyl)(methyl)amino)-4-methylpent-2-ynethioate | 11.31 μM |
| 18 | S-methyl 4-((2-hydroxyethyl)(methyl)amino)-4-methylpent-2-ynethioate | 6.75 μM |
| 19 | S-methyl 4-methyl-4-[methyl-[2-(2-naphthylmethoxy)ethyl]amino]pent-2-ynethioate | 11.31 μM |
| 20 | S-methyl 4-methyl-4-[methyl-[2-[(2,6,6-trimethylcyclohexen-1-yl)methoxy] ethyl]amino]pent -2-ynethioate | 13.62 μM |
| 21 | 2-[(1,1-dimethyl-4-methylsulfanyl-4-oxo-but-2-ynyl)-methylamino] ethyl acetate | 12.72 μM |
| 22 | 2-[(1,1-dimethyl-4-methylsulfanyl-4-oxo-but-2-ynyl)-methylamino] ethyl-3,4-dimeth oxybenzoate | 15.32 μM |
| 23 | S-methyl 2,5,10,11,11-pentamethyl-6-oxo-7-oxa-2,5,10-triazatetradec-12-yne-14-thioate | 10.61 μM |
| 24 | S-methyl 4-[2-(methoxymethyl)pyrrolidin-1-yl]-4-methylpent-2-ynethioate | 20.5 μM |
| 25 | S-methyl 4-(3-methoxypyrrolidin-1-yl)-4-methylpent-2-ynethioate | 16.5 μM |
| 26 | S-methyl 4-methyl-4-[methyl(2-phenoxycyclopentyl)amino]pent -2-ynethioate | 17.5 μM |
| 27 | (S)-S-methyl 4-(2-((benzyloxy)methyl)pyrrolidin-1-yl)-4-methylpent-2-yneth ioate | 10.38 μM |
| 28 | S-methyl 4-[3(benzyloxy)-1pyrrolidinyl])-4-methylpent-2-ynethioate | 11.83 μM |

**Example 2: activity of compounds 3 and 5 in enzymatic assays with human recombinant ALDH1A1, ALDH1A2, ALDH1A3, ALDH2 and ALDH3A1**

*Material and Methods*

Human recombinant ALDH1A1, ALDH1A2, ALDH1A3, ALDH2 (Creative BioMart, NY, USA) were prepared at 1mg/mL. The enzymatic reactions were performed using saturating concentrations of substrate. To test the enzymatic activity of the enzymes, 10 μL of enzyme was added into a reaction buffer containing 50 mM HEPES pH 7.2, 30 mM $MgCl_2$, plus 20 mM $NAD^+$ cofactor and 2 mM Hexanal (Sigma-Aldrich, St. Louis, Missouri, USA) in the presence or absence of the different tested compounds. Internal standards were prepared with Nicotinamide adenine dinucleotide reduce form (NADH, 500 μM, Sigma-Aldrich) in Reaction Buffer (50 mM HEPES pH 7.2, 30 mM $MgCl_2$).

For ALDH3A1 (1mg/mL), 10 μL of enzyme was added into a reaction buffer containing 50 mM Tris, 5 mM DTT, pH 8, plus 40 mM Nicotinamide adenine dinucleotide phosphate (oxidized form, $NADP^+$) and 4-Nitrobenzaldehyde (4-NBA) (Sigma-.Aldrich). Internal standards were prepared with Nicotinamide adenine dinucleotide phosphate reduced form (NADPH,5 μM, Sigma-Aldrich) in Reaction Buffer (50 mM Tris, 5 mM DTT, pH 8). Time-dependent inhibition assays were performed for 0-2-5-10-15-20-30-45-60 min at 37◦C in 1mL- quartz cuvette. The formation of NADH was monitored, reading samples at excitation wavelength = 340 nm/emission wavelength = 460 nm (fluorescence) for at least 600 sec using Cary Eclipse Varian fluorimeter.

Negative control consisted in the same reactions except that enzyme was not added (enzyme blank). To determine the slope for enzyme blank and calculate product concentration (Unit of fluorescence) the following formulae was used:

$$v = \frac{dF}{dt} x \frac{Cst}{Fst}$$

where *Cst* is the standard NADH concentration, *Fst* is the standard fluorescence and *dF/dt* is the slope of the time dependent fluorescence (S. Sotꞌoꞁobodowska et al, 2012).

The specific activity of the enzymes (μmol/min·mg - U/mg) in the absence of presence of inhibitors was calculated as follows:

$$\text{Specific Activity} = \frac{\text{Adjusted slope (μmols NADH/sec) } x \text{ time (60 sec/min) } x \text{ dilution factor}}{\text{Final volume assay (1000μL) } x \text{ enzyme volume (0.01 μL)} x \text{ enzyme concentration(mg/ml)}}$$

$$\text{Specific Activity} = \frac{\text{Adjusted slope (μmols NADPH/}sec\text{) } x \text{ time (60 sec/min) } x \text{ dilution factor}}{\text{Final volume assay (1000μL) } x \text{ enzyme volume (0.01 μL)} x \text{ enzyme concentration(mg/ml)}}$$

In said reactions, activity of compounds 3 and 5 according to the invention was compared to the one of DIMATE (S-methyl 4-(dimethylamino)-4-methylpent-2-ynethioate, described in EP 1296946).

*Results*

The $IC_{50}$ obtained are mentioned in table 4 below.

**Table 4**

| COMPOUND | ALDH1A1 $IC_{50}$ (μM) | ALDH1A2 $IC_{50}$ (μM) | ALDH1A3 $IC_{50}$ (μM) | ALDH3A1 $IC_{50}$ (μM) | ALDH2 $IC_{50}$ (μM) |
|---|---|---|---|---|---|
| DIMATE (not part of the invention) | 37 ± 5 | 18 ± 4 | 20 ± 2 | 303 ± 46 | 72 ± 9 |
| Compound 3 | 3.8 ± 1.1 | 0.568 ± 0.09 | 3.4 ± 0.1 | 242 ± 11 | 3.1 ± 0.8 |
| Compound 5 | 4.3 ± 0.4 | 1.3 ± 0.2 | 4.8 ± \0.1 | 143 ± 2 | 12 ± 3 |

Compounds 3 and 5 showed higher inhibition of the ALDH class 1 enzymes than that of DIMATE.

**Example 3: Kinetic parameters for compounds 3 and 5 in reactions with human recombinant ALDH1A1, ALDH1A2, ALDH1A3, ALDH2 or ALDH3A1**

***Material and Methods***

The kinetic data are expressed as the mean ± standard error from three independent determination. Kinact/Ki was determined from Kobs versus concentration of the inhibitor [I] plots. Kobs was determined from product concentration vs time incubation plot of the enzymes with compounds 3 and 5 or DIMATE at different concentration (i.e. 300 μM, 200 μM, 100 μM, 50 μM, 20 μM, and 10 μM of inhibitors). The Kobs were obtained from negative exponential fit using non-linear regression program GraFit 5.0 (Erithacus software).

***Results***

The kinetic parameters obtained are mentioned in table 5 below.

**Table 5**

| COMPOUND | ALDH1A1 K*inact*/K*i* ($M^{-1} \cdot min^{-1}$) | ALDH1A2 *Kinact*/ *Ki* ($M^{-1} \cdot min^{-1}$) | ALDH1A3 *Kinact*/ *Ki* ($M^{-1} \cdot min^{-1}$) | ALDH3A1 *Kinact*/ *Ki* ($M^{-1} \cdot min^{-1}$) | ALDH2 *Kinact*/*Ki* ($M^{-1} \cdot min^{-1}$) |
|---|---|---|---|---|---|
| DIMATE (not part of the invention) | 900 | 5 100 | 1 700 | *Ki* =253 ± 48 μM | 425 |
| Compound 3 | 500 | 100 000 | 38 500 | *Ki* =233 ± 16 μM | 540 |
| Compound 5 | 3 700 | 21 100 | 11 800 | 300 | 2450 |

The inhibitory potency of compounds 3 and 5 is between 4 and 20-fold higher than that of DIMATE for ALDH class 1 recombinant enzymes, in particular ALDH1A2 and ALDH1A3.

**Example 4: Inhibition type following full enzymatic and biochemical characterization of compounds 3 and 5**

***Material and Methods***

To determine the inhibition mechanisms of the tested compounds for each isoenzyme, the corresponding Kobs were calculated as mentioned above, plotting product concentration vs time incubation of the enzymes with the test compounds at 300 μM, 200 μM, 100 μM, 50 μM, 20 μM and 10 μM. The $k_{obs}$ were obtained for each concentration of inhibitor tested, from the negative exponential fit using non-linear regression program GraFit 5.0 (Erithacus software). Finally, the kinetic parameter of $k_{inact}/K_I$ was determined from the plot of $k_{obs}$ *versus* the corresponding concentration of inhibitor. The slope of the linear fit of data indicated the rate constant in $\mu M^{-1} \cdot min^{-1}$. Based on the different plots obtained, the irreversible inhibition was characterized as Specific or Non-Specific Affinity Labelling.

In case of Specific Affinity Labelling the plot exhibits a saturated kobs versus concentration of inhibitor diagram (similar to a reversible inhibition plot), achieving a plateau at high concentrations of inhibitor. In Non-Specific Affinity Labelling, the dependence of $k_{obs}$ on inhibitor concentration appears as non-saturating.

In said experiment, compounds 3 and 5 were compared to Dimate.

***Results***

The results obtained are mentioned in table 6 below.

**Table 6**

| COMPOUND | ALDH1A1 Inhibition Type | ALDH1A2 Inhibition Type | ALDH1A3 Inhibition Type | ALDH3A1 Inhibition Type | ALDH2 Inhibition Type |
|---|---|---|---|---|---|
| DIMATE | specific | Non-specific affinity label | Non-specific affinity label | Noncompetitive | Non-specific affinity label |
| Compound 3 | Non-specific affinity label | Non-specific affinity label | specific | Noncompetitive | Non-specific affinity label |
| Compound 5 | Non-specific affinity label | Non-specific affinity label | specific | Non-specific affinity label | Non-specific affinity label |

Although all the characterized compounds showed irreversible inhibition, the type of inactivation observed for the different isoenzymes varied between specific and non-specific affinity label. Notably, compounds 3 and 5 interact with higher specificity with ALDH1A3 while for DIMATE that inhibition takes place by a single-step mechanism of inactivation as described for non-specific affinity label, irreversible inhibitors.

## Part 3: Preparation of an antibody drug conjugate (ADC) according to the invention

ADC is a three-components system including a cytotoxic agent linked via a biodegrable linker to an antibody. The antibody binds to specific markers (antigens or receptors) at the surface if the cancer cell. The whole antibody-drug conjugate is then internalized within the cancer cell, where the linker is degraded and the active drug released.

In the context of the present invention, cytotoxic agent, a compound according to the invention, is coupled to antibody via an attachment group (maleimide, succinimidyl ester, specific peptidic sequence substrate of enzyme, etc ...), linked to a cleavable linker (protease site, hydrazine, disulfide) or non-cleavable and with or not a self-imolative spacer.

### Synthesis of the compound according to the invention

Reference is made to part 1 example 29.

### Conjugation with the antibody rituximab

Antibody rituximab (Roche® was mixed with DTT at 37°C for 30 minutes and then diafiltered against PBS containing 1 mmol/L EDTA using Amicon Ultra-15, MWCO 30kDa, Merck-Millipore. The thiol concentration was quantified by Ellman's reagent, 5,5'-dithiobis(2-nitrobenzoic acid) [DTNB]. A 50-fold molar excess of the final compound 9 obtained in the precedent paragraph dissolve in DMF, was added to the reduced antibody at 4°C for 1 hour. Antibody-Drug conjugate was diafiltered in PBSx1 using Amicon Ultra-15, MWCO 30kDa, Merck-Millipore. For the determination of Drug Antibody Ratio (DAR), the thiol concentration of modified antibody after coupling was quantified by Ellman's reagent.

The mechanism of release of the compound according to the invention followed by the cathepsin cleavage group is shown in the scheme below:

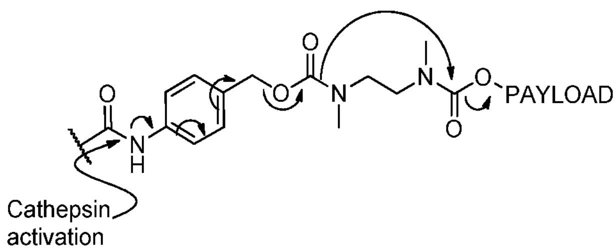


Released fragments :

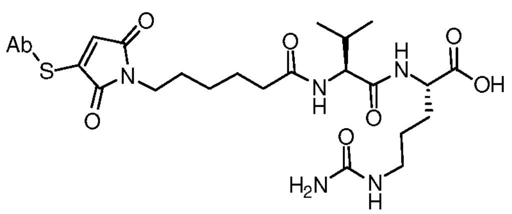

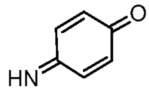

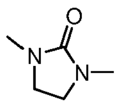

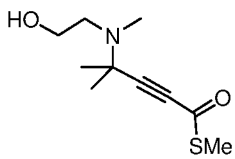

## Part 4: Use of an ADC according to the invention

### Drugs/Antibody Ratio (DAR) Determination.

DAR of Antibody-Drug Conjugate mentioned in part 3 was controlled by the difference between the thiol quantification using Ellman's Reagent, after the mild thiolation of rituximab by dithiothreitol (DTT) and the quenching of these free thiol by the coupling of the maleimide group. After DTT thiolation, 10 new free thiol group were produced by Rituximab molecule. After coupling of the final product 9, the totality of these new free thiol group was quenched resulting a coupling of 10 compounds 9 per Rituximab molecule.

### Cell Cytoxicity by Rituximab-compound 9.

50 000 viable Raji cells were plated in triplicate. Then, serial 1:2 dilutions of Rituximab-compound 9 or a control Rituximab were added to yield the final concentrations (starting concentrations 50μg/mL). The cells were incubated for 48h at which time 20 μl of Alamar Blue (Thermo Fisher Scientific) was added to each well. The plates were incubated

for an additional four hours and the fluorescence intensity read on a plate reader using excitation wavelength of 540 nm and an emission wavelength of 620 nm.

The results show that Raji cells viability was significantly (Figure 1; p-value < 0.01; **) less using a 500 μg/ml treatment of Rituximab-compound 9 for 48 hours than Rituximab *per se.*

## Claims

1. A compound of formula (I) :

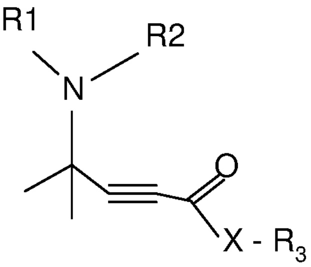

(I)

In which:

- X is S;
- R1 is linear or branched ($C_1$-$C_7$)alkyl,
- R2 is $CHR_5CHR_6OR_4$ or $(CHR_5)_vOR_4$,
- v is chosen from 2 to 4;
- $R_3$ is linear or branched ($C_1$-$C_7$)alkyl;
- $R_4$ is chosen from: H, linear or branched ($C_2$-$C_7$)alkyl, linear or branched ($C_2$-C7)alkenyl, -$CONR_7R_8$, aryl, heteroaryl, ($C_2$-$C_7$)cycloalkyl, linear or branched -($C_1$-$C_7$)alkyl-aryl and linear or branched -($C_1$-$C_7$)alkyl-heteroaryl;
said aryl, ($C_2$-$C_7$)cycloalkyl, and heteroaryl being optionally substituted by one or more substituents chosen from: halogen, linear or branched ($C_1$-$C_7$)alkyl optionally substituted by one or more halogen atom, linear or branched ($C_1$-$C_7$)alkoxy optionally substituted by one or more halogen atom, -COOH, aryl, -NRR', -$NO_2$, or said aryl and heteroaryl being optionally fused to form an heterocycloalkyl;
- $R_5$ and $R_6$ are chosen from:
    • H, or
    • $R_5$ is H and R1 and $R_6$ are linked together to form with the nitrogen atom linked to R1 an heterocycloalkyl, or
    • $R_6$ is H and R1 and $R_5$ are linked together to R1 to form with the nitrogen atom linked to R1 an heterocycloalkyl;
- $R_7$ is -($C_1$-$C_3$)alkyl;
- $R_8$ is -($C_1$-$C_3$)alkylNRR';
- R and R' identical or different, are independently chosen from H and linear or branched ($C_1$-$C_7$)alkyl,

or its pharmaceutically acceptable salts or optical isomers, racemates, diastereoisomers, enantiomers or tautomers.

2. A compound according to claim 1 in which R3 is methyl, R1 is methyl, R2 is $CHR_5CHR_6OR_4$ or $(CHR_5)_vOR_4$ and R5 and R6 are:

- H, or
- $R_5$ is H and R1 and $R_6$ are linked together to form with the nitrogen atom linked to R1 a pyrrolidinyl, or
- $R_6$ is H and R1 and $R_5$ are linked together to R1 to form with the nitrogen atom linked to R1 a pyrrolidinyl.

3. A compound according to claim 1 or 2, in which $R_4$ is chosen from linear or branched (C2-C7)alkyl, linear or branched (C2-C7)alkenyl, -$CONR_7R_8$, ($C_2$-$C_7$)cycloalkyl, linear or branched -($C_1$-$C_7$)alkyl-heteroaryl, aryl, or benzyl; said ($C_2$-$C_7$) cycloalkyl being substituted by one or more substituents chosen from: linear or branched (C1-$C_7$)alkyl, said

benzyl being optionally substituted by one or more substituents chosen from: linear or branched ($C_1$-$C_7$)alkyl optionally substituted by one or more halogen atom, linear or branched ($C_1$-$C_7$)alkoxy optionally substituted by one or more halogen atom, halogen or said benzyl being optionally fused to form 1,3-benzodioxole.

**4.** A compound according to claim 1 or 2, in which $R_5$ and $R_6$ are H and $R_4$ is chosen from linear or branched ($C_2$-$C_7$)alkyl, linear or branched ($C_2$-$C_7$)alkenyl,-CONR7$R_8$, ($C_2$-$C_7$) cycloalkyl, linear or branched -($C_1$-$C_7$)alkyl-heteroaryl, or benzyl; said ($C_2$-$C_7$) cycloalkyl being substituted by one or more substituents chosen from: linear or branched ($C_1$-$C_7$)alkyl, said benzyl being optionally substituted by one or more substituents chosen from: linear or branched (C1-$C_7$)alkyl optionally substituted by one or more halogen atom, linear or branched ($C_1$-$C_7$)alkoxy optionally substituted by one or more halogen atom, halogen.

**5.** A compound according to claim 4, in which R1 is methyl and $R_4$ is chosen from: , -CONR7$R_8$ with R7 being a methyl and $R_8$ being NRR' with R and R' being methyl, ethyl, propenyl, benzyl, pyridyl, benzyloxybutyl, methyl-cyclohexenyl substituted by one or more methyl, and benzyl substituted by one of more fluorine, chlorine, methoxy or methyl.

**6.** The compound according to any one of claims 1 to 5, chosen in the group consisting of:

- S-methyl 4-[2-ethoxyethyl(methyl)amino]-4-methyl-pent-2-ynethioate;
- S-methyl 4-[2-allyloxyethyl(methyl)amino]-4-methyl-pent-2-ynethioate;
- S-methyl 4-[2-benzyloxyethyl(methyl)amino]-4-methyl-pent-2-ynethioate;
- S-methyl 4-methyl-4-[methyl-[2-(m-tolylmethoxy)ethyl]amino]pent-2-ynethioate;
- S-methyl 4-[2-[(3,4-dimethylphenyl)methoxy]ethyl-methyl-amino]-4-methyl-pent-2-ynethioate;
- S-methyl 4-[2-[(4-methoxyphenyl)methoxy]ethyl-methyl-amino]-4-methyl-pent-2-ynethioate;
- S-methyl 4-[2-[(3,4-dimethoxyphenyl)methoxy]ethyl-methyl-amino]-4-methyl-pent-2-ynethioate;
- S-methyl 4-[2-[(3-chlorophenyl)methoxy]ethyl-methyl-amino]-4-methyl-pent-2-ynethioate;
- S-methyl 4-[2-[(3-fluorophenyl)methoxy]ethyl-methyl-amino]-4-methyl-pent-2-ynethioate;
- S-methyl 4-methyl-4-[methyl-[2-(2-pyridylmethoxy)ethyl]amino]pent-2-ynethioate;
- S-methyl 4-methyl-4-[methyl-[2-(3-pyridylmethoxy)ethyl]amino]pent-2-ynethioate;
- S-methyl 4-methyl-4-[methyl-[2-(4-pyridylmethoxy)ethyl]amino]pent-2-ynethioate;
- S-methyl 4-((4-(benzyloxy)butyl)(methyl)amino)-4-methylpent-2-ynethioate;
- S-methyl 4-((2-hydroxyethyl)(methyl)amino)-4-methylpent-2-ynethioate;
- S-methyl 4-methyl-4-[methyl-[2-(2-naphthylmethoxy)ethyl]amino]pent-2-ynethioate;
- S-methyl 4-methyl-4-[methyl-[2-[(2,6,6-trimethylcyclohexen-1-yl)methoxy]ethyl]amino]pent-2-ynethioate;
- 2-[(1,1-dimethyl-4-methylsulfanyl-4-oxo-but-2-ynyl)-methylamino] ethyl-3,4-dimethoxybenzoate;
- 2[(1,1-dimethyl-4-methylsulfanyl-4-oxo-but-2-ynyl)-methylamino] ethyl acetate;
- S-methyl 2,5,10,11,11-pentamethyl-6-oxo-7-oxa-2,5,10-triazatetradec-12-yne-14-thioate;
- S-methyl 4-[2-(methoxymethyl)pyrrolidin-1-yl]-4-methylpent-2-ynethioate;
- S-methyl 4-(3-methoxypyrrolidin-1-yl)-4-methylpent-2-ynethioate;
- S-methyl 4-methyl-4-[methyl(2-phenoxycyclopentyl)amino]pent-2-ynethioate;
- (S)-S-methyl 4-(2-((benzyloxy)methyl)pyrrolidin-1-yl)-4-methylpent-2-ynethioate;
- S-methyl 4-[3(benzyloxy)-1pyrrolidinyl])-4-methylpent-2-ynethioate

or its pharmaceutically acceptable salts or optical isomers, racemates, diastereoisomers, enantiomers or tautomers.

**7.** A compound according to claim 1, in which:

- X is S;
- R1 is linear or branched ($C_1$-$C_7$)alkyl;
- R2 is $CHR_5CHR_6OR_4$ or $(CHR_5)_yOR_4$;
- R4 is chosen from H, aryl, heteroaryl, linear or branched -($C_1$-$C_7$)alkyl-aryl and linear or branched -($C_1$-$C_7$)alkyl-heteroaryl;
said aryl and heteroaryl being optionally substituted by one or more substituents chosen from: -COOH, -NRR' and -$NO_2$; and
- R and R' identical, are H.

**8.** A process for preparing a compound according to any of claims 1 to 7, comprising :

a) reacting a compound of formula (II) with an organic or inorganic acid

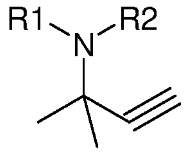

(II)

b) reacting the compound obtained in step a) with a base ;
c) reacting the compound obtained in step b) with $CO_2$ ;
d) reacting the compound obtained in step c) with alkyl chloroformate, a reagent able of forming, with the compound obtained in step c), an acid halide or a reagent able of forming, with the compound obtained in step c), a mixed anhydride ;
e) reacting the compound obtained in step d) with an anion precursor compound $SMe^-$,

wherein R1 and R2 are defined as in any of claims 1 to 7.

**9.** A pharmaceutical composition comprising a compound according to any of claims 1 to 7 and a pharmaceutical acceptable excipient.

**10.** A compound according to any of claims 1 to 7 for use as a medicament.

**11.** A compound according to any of claims 1 to 7 for use for the prevention or treatment of cancer, in particular leukemia.

**12.** An antibody drug conjugate of formula: B-L-Ab, wherein:

- B is a compound of formula (I) as defined in claim 7;
- L is a chemical moiety comprising a covalent bond or a chain of atoms that covalently attaches the Ab to the compound of formula (I); and
- Ab is an antibody chosen from: rituximab, trastuzumab, alemtuzumab, ibritumomab, gemtuzumab,tiuxetan, tositumomab, brevacizumab, cetuximab, panitumumab, ofatumumab and obinutuzumab.

**Patentansprüche**

**1.** Verbindung von Formel (I):

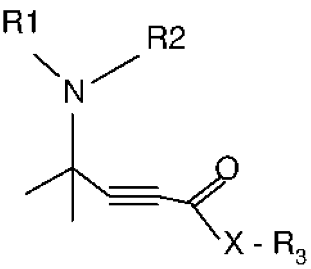

(I)

Wobei:

- X S ist;
- R1 lineares oder verzweigtes $(C_1-C_7)$-Alkyl ist,
- R2 $CHR_5CHR_6OR_4$ oder $(CHR_5)_vOR_4$ ist,
- v ausgewählt ist aus 2 bis 4;
- $R_3$ lineares oder verzweigtes $(C_1-C_7)$-Alkyl ist;
- $R_4$ ausgewählt ist aus: H, linearem oder verzweigtem $(C_2-C_7)$-Alkyl, linearem oder verzweigtem $(C_2-C_7)$-Alkenyl, $-CONR_7R_8$, Aryl, Heteroaryl, $(C_2-C_7)$-Cycloalkyl, linearem oder verzweigtem $-(C_1-C_7)$-Alkylaryl und linearem oder verzweigtem $-(C_1-C_7)$-Alkylheteroaryl;
wobei das Aryl, $(C_2-C_7)$-Cycloalkyl und Heteroaryl optional durch einen oder mehrere Substituenten substituiert sind, die ausgewählt sind aus: Halogen, linearem oder verzweigtem $(C_1-C_7)$-Alkyl, das optional durch ein oder

mehrere Halogenatome substituiert ist, linearem oder verzweigtem ($C_1$-$C_7$)-Alkoxy, das optional durch ein oder mehrere Halogenatome substituiert ist, -COOH, Aryl, -NRR', -$NO_2$, oder wobei das Aryl und das Heteroaryl optional kondensiert sind, um ein Heterocycloalkyl zu bilden;

- $R_5$ und $R_6$ ausgewählt sind aus:

  • H,oder
  • $R_5$ H ist und R1 und $R_6$ miteinander verbunden sind, um mit dem an R1 gebundenen Stickstoffatom ein Heterocycloalkyl zu bilden, oder
  • $R_6$ H ist und R1 und $R_5$ mit R1 verbunden sind, um mit dem an R1 gebundenen Stickstoffatom ein Heterocycloalkyl zu bilden;

- $R_7$ -($C_1$-$C_3$)-Alkyl ist;
- $R_8$ -($C_1$-$C_3$)-AlkylNRR' ist;
- R und R', die gleich oder verschieden sind, unabhängig ausgewählt sind aus H und linearem oder verzweigtem ($C_1$-$C_7$)-Alkyl,

oder seine pharmazeutisch annehmbaren Salze oder optischen Isomere, Racemate, Diastereoisomere, Enantiomere oder Tautomere.

**2.** Verbindung nach Anspruch 1, wobei R3 Methyl ist, R1 Methyl ist, R2 $CHR_5CHR_6OR_4$ oder $(CHR_5)_vOR_4$ ist und R5 und R6 wie folgt sind:

- H,oder
- $R_5$ H ist und R1 und $R_6$ miteinander verbunden sind, um mit dem an R1 gebundenen Stickstoffatom ein Pyrrolidinyl zu bilden, oder
- $R_6$ H ist und R1 und $R_5$ miteinander an R1 gebunden sind, um mit dem an R1 gebundenen Stickstoffatom ein Pyrrolidinyl zu bilden.

**3.** Verbindung nach Anspruch 1 oder 2, wobei $R_4$ ausgewählt ist aus linearem oder verzweigtem ($C_2$-$C_7$)-Alkyl, linearem oder verzweigtem ($C_2$-$C_7$)-Alkenyl, -CONR7$R_8$, (C2-C7)-Cycloalkyl, linearem oder verzweigtem -($_{C1-C7}$)-Alkylheteroaryl, Aryl oder Benzyl; wobei das ($C_2$-$C_7$)-Cycloalkyl durch einen oder mehrere Substituenten substituiert ist, ausgewählt aus: linearem oder verzweigtem ($C_1$-$C_7$)-Alkyl, wobei das Benzyl optional durch einen oder mehrere Substituenten substituiert ist, die ausgewählt sind aus: linearem oder verzweigtem ($C_1$-$C_7$)-Alkyl, das optional durch ein oder mehrere Halogenatome substituiert ist, linearem oder verzweigtem ($C_1$-$C_7$)-Alkoxy, das optional durch ein oder mehrere Halogenatome substituiert ist, Halogen oder wobei das Benzyl optional kondensiert ist, um 1,3-Benzodioxol zu bilden.

**4.** Verbindung nach Anspruch 1 oder 2, wobei $R_5$ und $R_6$ H sind und R4 ausgewählt ist aus linearem oder verzweigtem ($C_2$-$C_7$)-Alkyl, linearem oder verzweigtem ($C_2$-$C_7$)-Alkenyl, -$CONR_7R_8$, ($C_2$-$C_7$)-Cycloalkyl, linearem oder verzweigtem -($C_1$-$C_7$)-Alkylheteroaryl oder Benzyl; wobei das ($C_2$-$C_7$)-Cycloalkyl durch einen oder mehrere Substituenten substituiert ist, ausgewählt aus: linearem oder verzweigtem ($C_1$-$C_7$)-Alkyl, wobei das Benzyl optional substituiert ist durch einen oder mehrere Substituenten, ausgewählt aus: linearem oder verzweigtem ($C_1$-$C_7$)-Alkyl, optional substituiert durch ein oder mehrere Halogenatome, linearem oder verzweigtem ($C_1$-$C_7$)-Alkoxy, optional substituiert durch ein oder mehrere Halogenatome, Halogen.

**5.** Verbindung nach Anspruch 4, wobei R1 Methyl ist und $R_4$ ausgewählt ist aus: -$CONR_7R_8$, wobei R7 eine Methylgruppe ist und $R_8$ NRR' ist, wobei R und R' Methyl, Ethyl, Propenyl, Benzyl, Pyridyl, Benzyloxybutyl, Methyl-Cyclohexenyl, substituiert mit einer oder mehreren Methylgruppen, und Benzyl, substituiert mit einem oder mehreren Fluor-, Chlor-, Methoxy- oder Methylgruppen, sind.

**6.** Verfahren nach einem der Ansprüche 1 bis 5, ausgewählt aus der Gruppe, bestehend aus:

- S-Methyl-4-[2-ethoxyethyl(methyl)amino]-4-methyl-pent-2-ynethioat;
- S-Methyl-4-[2-allyloxyethyl(methyl)amino]-4-methyl-pent-2-ynethioat;
- S-Methyl-4-[2-benzyloxyethyl(methyl)amino]-4-methyl-pent-2-ynethioat;
- S-Methyl-4-methyl-4-[methyl-[2-(m-tolylmethoxy)ethyl]amino]pent-2-ynethioat;
- S-Methyl-4-[2-[(3,4-dimethylphenyl)methoxy]ethyl-methyl-amino]-4-methyl-pent-2-ynethioat;
- S-Methyl-4-[2-[(4-methoxyphenyl)methoxy]ethyl-methyl-amino]-4-methyl-pent-2-ynethioat;
- S-Methyl-4-[2-[(3,4-dimethoxyphenyl)methoxy]ethyl-methyl-amino]-4-methyl-pent-2-ynethioat;

- S-Methyl-4-[2-[(3-chlorphenyl)methoxy]ethyl-methyl-amino]-4-methyl-pent-2-ynethioat;
- S-Methyl-4-[2-[(3-fluorphenyl)methoxy]ethyl-methyl-amino]-4-methyl-pent-2-ynethioat;
- S-Methyl-4-methyl-4-[methyl-[2-(2-pyridylmethoxy)ethyl]amino]pent-2-ynethioat;
- S-Methyl-4-methyl-4-[methyl-[2-(3-pyridylmethoxy)ethyl]amino]pent-2-ynethioat;
- S-Methyl-4-methyl-4-[methyl-[2-(4-pyridylmethoxy)ethyl]amino]pent-2-ynethioat;
- S-Methyl-4-((4-(benzyloxy)butyl)(methyl)amino)-4-methylpent-2-ynethioat;
- S-Methyl-4-((2-hydroxyethyl)(methyl)amino)-4-methylpent-2-ynethioat;
- S-Methyl-4-methyl-4-[methyl-[2-(2-naphthylmethoxy)ethyl]amino]pent-2-ynethioat;
- S-Methyl-4-methyl-4-[methyl-[2-[(2,6,6-trimethylcyclohexen-1-yl)methoxy]ethyl]am ino]pent-2-ynethioat;
- 2-[(1,1-Dimethyl-4-methylsulfanyl-4-oxo-but-2-ynyl)-methylamino]ethyl-3,4-dimethoxybenzoat;
- 2[(1,1 -Dimethyl-4-methylsulfanyl-4-oxo-but-2-ynyl)-methylamino]ethylacetat;
- S-Methyl-2,5,10,11,11-pentamethyl-6-oxo-7-oxa-2,5,10-triazatetradec-12-yne-14-thioat;
- S-Methyl-4-[2-(methoxymethyl)pyrrolidin-1-yl]-4-methylpent-2-ynethioat;
- S-Methyl-4-(3-methoxypyrrolidin-1-yl)-4-methylpent-2-ynethioat;
- S-Methyl-4-methyl-4-[methyl(2-phenoxycyclopentyl)amino]pent-2-ynethioat;
- (S)-S-Methyl-4-(2-((benzyloxy)methyl)pyrrolidin-1-yl)-4-methylpent-2-ynethioat;
- S-Methyl-4-[3(benzyloxy)-1pyrrolidinyl])-4-methylpent-2-ynethioat

oder seine pharmazeutisch annehmbaren Salze oder optischen Isomere, Racemate, Diastereoisomere, Enantiomere oder Tautomere.

**7.** Verbindung nach Anspruch 1, wobei:

- X S ist;
- R1 lineares oder verzweigtes ($C_1$-$C_7$)-Alkyl ist;
- R2 $CHR_5CHR_6OR_4$ oder $(CHR_5)_vOR_4$ ist;
- R4 ist ausgewählt aus H, Aryl, Heteroaryl, linearem oder verzweigtem -($C_1$-$C_7$)-Alkylaryl und linearem oder verzweigtem -($C_1$-$C_7$)-Alkylheteroaryl;
wobei das Aryl und das Heteroaryl optional durch einen oder mehrere Substituenten substituiert sind, ausgewählt aus: -COOH, -NRR' und -$NO_2$; und
- R und R', die identisch sind, H sind.

**8.** Verfahren zur Herstellung einer Verbindung nach einem der Ansprüche 1 bis 7, umfassend:

a) Reagieren einer Verbindung von Formel (II) mit einer organischen oder anorganischen Säure

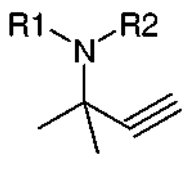

(II)

b) Reagieren der in Schritt a) erlangten Verbindung mit einer Base;
c) Reagieren der in Schritt b) erlangten Verbindung mit $CO_2$;
d) Reagieren der in Schritt c) erlangten Verbindung mit Chlorameisensäurealkylester, einem Reagenz, das mit der in Schritt c) erlangten Verbindung ein Säurehalogenid bilden kann, oder einem Reagenz, das mit der in Schritt c) erlangten Verbindung ein gemischtes Anhydrid bilden kann;
e) Reagieren der in Schritt d) erlangten Verbindung mit einer Anionenvorläuferverbindung SMe;

wobei R1 und R2 wie definiert in einem der Ansprüche 1 bis 7 sind.

**9.** Pharmazeutische Zusammensetzung, umfassend eine Verbindung nach einem der Ansprüche 1 bis 7 und einen pharmazeutisch annehmbaren Träger.

**10.** Eine Verbindung nach einem der Ansprüche 1 bis 7 zur Verwendung als Medikament.

**11.** Verbindung nach einem der Ansprüche 1 bis 7 zur Verwendung bei der Prävention oder Behandlung von Krebs,

insbesondere Leukämie.

**12.** Antikörper-Arzneimittelkonjugat von Formel: B-L-Ab, wobei:

- B eine Verbindung von Formel (I) ist, wie es in Anspruch 7 definiert ist;
- L eine chemische Einheit ist, umfassend eine kovalente Bindung oder eine Kette von Atomen, die das Ab kovalent an die Verbindung von Formel (I) anlagert; und
- Ab einen Antikörper ist, ausgewählt aus: Rituximab, Trastuzumab, Alemtuzumab, Ibritumomab, Gemtuzumab, Tiuxetan, Tositumomab, Brevacizumab, Cetuximab, Panitumumab, Ofatumumab und Obinutuzumab.

**Revendications**

**1.** Composé de la formule (I) :

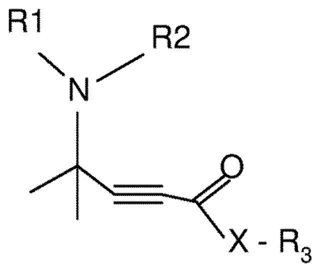

(I)

dans laquelle :

- X représente S ;
- R1 représente un alkyle en $C_1$-$C_7$ linéaire ou ramifié,
- R2 représente $CHR_5CHR_6OR_4$ ou $(CHR_5)_vOR_4$,
- $_v$ est compris entre 2 à 4 ;
- $R_3$ représente un alkyle en $C_1$-$C_7$ linéaire ou ramifié ;
- $R_4$ est choisi parmi : H, alkyle en $C_2$-$C_7$ linéaire ou ramifié, alcényle en $C_2$-$C_7$ linéaire ou ramifié, -$CONR_7R_8$, aryle, hétéroaryle, cycloalkyle en C2-C7, alkyl-aryle en $C_1$-$C_7$ linéaire ou ramifié et alkyl-hétéroaryle en $C_1$-$C_7$ linéaire ou ramifié ; lesdits aryle, cycloalkyle en $C_2$-$C_7$ et hétéroaryle étant éventuellement substitués par un ou plusieurs substituants choisis parmi : un halogène, un alkyle en $C_1$-$C_7$ linéaire ou ramifié éventuellement substitué par un ou plusieurs atomes d'halogène, un alcoxy en $C_1$-$C_7$ linéaire ou ramifié éventuellement substitué par un ou plusieurs atomes d'halogène, - COOH, un aryle, -NRR', -$NO_2$, ou lesdits aryle et hétéroaryle étant éventuellement fusionnés pour former un hétérocycloalkyle ;
- $R_5$ et $R_6$ sont choisi parmi :

  • H, ou
  • $R_5$ représente H et R1 et $R_6$ sont liés ensemble pour former un hétérocycloalkyle avec l'atome d'azote lié à R1, ou
  • $R_6$ représente H et R1 et $R_5$ sont liés ensemble à R1 pour former un hétérocycloalkyle avec l'atome d'azote lié à R1 ;

- $R_7$ représente un alkyle en $C_1$-$C_3$ ;
- $R_8$ représente un alkylNRR' en $C_1$-$C_3$ ;
- R et R' identiques ou différents, sont indépendamment choisis parmi H et un alkyle en $C_1$-$C_7$ linéaire ou ramifié,

ou ses sels pharmaceutiquement acceptables ou ses isomères optiques, racémates, diastéréoisomères, énantiomères ou tautomères.

**2.** Composé selon la revendication 1, dans lequel R3 représente un méthyle, R1 représente un méthyle, R2 représente $CHR_5CHR_6OR_4$ ou $(CHR_s)_vOR_4$ et R5 et R6 représentent :

- H, ou
- $R_5$ représente H et R1 et $R_6$ sont liés ensemble pour former un pyrrolidinyle avec l'atome d'azote lié à R1, ou
- $R_6$ représente H et R1 et $R_5$ sont liés ensemble à R1 pour former un pyrrolidinyle avec l'atome d'azote lié à R1.

**3.** Composé selon la revendication 1 ou 2, dans lequel R4 est choisi parmi un alkyle en $C_2$-$C_7$ linéaire ou ramifié, un alcényle en $C_2$-$C_7$ linéaire ou ramifié, -CON$R_7R_8$, un cycloalkyle en $C_2$-$C_7$, un alkyl-hétéroaryle en $C_1$-$C_7$ linéaire ou ramifié, un aryle et un benzyle ; ledit cycloalkyle en $C_2$-$C_7$ étant substitué par un ou plusieurs substituants choisis parmi : un alkyle en $C_1$-$C_7$ linéaire ou ramifié, ledit benzyle étant éventuellement substitué par un ou plusieurs substituants choisis parmi : un alkyle en $C_1$-$C_7$ linéaire ou ramifié éventuellement substitué par un ou plusieurs atomes d'halogène, un alcoxy en $C_1$-$C_7$ linéaire ou ramifié éventuellement substitué par un ou plusieurs atomes d'halogène, un halogène ou ledit benzyle étant éventuellement fusionné pour former du 1,3-benzodioxole.

**4.** Composé selon la revendication 1 ou 2, dans lequel $R_5$ et $R_6$ représentent H et $R_4$ est choisi parmi un alkyle en $C_2$-$C_7$ linéaire ou ramifié, un alcényle en $C_2$-$C_7$ linéaire ou ramifié, -CON$R_7R_8$, un cycloalkyle en $C_2$-$C_7$, un alkyl-hétéroaryle en $C_1$-$C_7$ linéaire ou ramifié et un benzyle ; ledit cycloalkyle en $C_2$-$C_7$ étant substitué par un ou plusieurs substituants choisis parmi : un alkyle en $C_1$-$C_7$ linéaire ou ramifié, ledit benzyle étant éventuellement substitué par un ou plusieurs substituants choisis parmi : un alkyle en $C_1$-$C_7$ linéaire ou ramifié éventuellement substitué par un ou plusieurs atomes d'halogène, un alcoxy en $C_1$-$C_7$ linéaire ou ramifié éventuellement substitué par un ou plusieurs atomes d'halogène, et un halogène.

**5.** Composé selon la revendication 4, dans lequel R1 représente un méthyle et $R_4$ est choisi parmi :

- CON$R_7R_8$, R7 représentant un méthyle et $R_8$ représentant NRR', R et R' représentant un méthyle, un éthyle, un propényle, un benzyle, un pyridyle, un benzyloxybutyle, un méthyl-cyclohexényle substitué par un ou plusieurs méthyles, et un benzyle substitué par un ou plusieurs fluors, chlores, méthoxy ou méthyles.

**6.** Composé selon l'une quelconque des revendications 1 à 5, choisi dans le groupe constitué par :

- 4-[2-éthoxyéthyl(méthyl)amino]-4-méthyl-pent-2-ynethioate de S-méthyle ;
- 4-[2-Allyloxyéthyl(méthyl)amino]-4-méthyl-pent-2-ynethioate de S-méthyle ;
- 4-[2-Benzyloxyéthyl(méthyl)amino]-4-méthyl-pent-2-ynethioate de S-méthyle ;
- 4-méthyl-4-[méthyl-[2-(m-tolylméthoxy)éthyl]amino]pent-2-ynethioate de S-méthyle ;
- 4-[2-[(3,4-diméthylphényl)méthoxy]éthyl-méthyl-amino]-4-méthyl-pent-2-ynethioate de S-méthyle ;
- 4-[2-[(4-méthoxyphényl)méthoxy]éthyl-méthyl-amino]-4-méthyl-pent-2-ynethioate de S-méthyle ;
- 4-[2-[(3,4-diméthoxyphényl)méthoxy]éthyl-méthyl-amino]-4-méthyl-pent-2-ynethioate de S-méthyle ;
- 4-[2-[(3-chlorophényl)méthoxy]éthyl-méthyl-amino]-4-méthyl-pent-2-ynethioate de S-méthyle ;
- 4-[2-[(3-fluorophényl)méthoxy]éthyl-méthyl-amino]-4-méthyl-pent-2-ynethioate de S-méthyle ;
- 4-méthyl-4-[méthyl-[2-(2-pyridylméthoxy)éthyl]amino]pent-2-ynethioate de S-méthyle ;
- 4-méthyl-4-[méthyl-[2-(3-pyridylméthoxy)éthyl]amino]pent-2-ynethioate de S-méthyle ;
- 4-méthyl-4-[méthyl-[2-(4-pyridylméthoxy)éthyl]amino]pent-2-ynethioate de S-méthyle ;
- 4-((4-(Benzyloxy)butyl)(méthyl)amino)-4-méthylpent-2-ynethioate de S-méthyle ;
- 4-((2-Hydroxyéthyl)(méthyl)amino)-4-méthylpent-2-ynethioate de S-méthyle ;
- 4-méthyl-4-[méthyl-[2-(2-naphtylméthoxy)éthyl]amino]pent-2-ynethioate de S-méthyle ;
- 4-méthyl-4-[méthyl-[2-[(2,6,6-triméthylcyclohexèn-1-yl)méthoxy]éthyl]amino]pent-2-ynethioate de S-méthyle ;
- 2-[(1,1-diméthyl-4-méthylsulfanyl-4-oxo-but-2-ynyl)-méthylamino] éthyl-3,4-diméthoxybenzoate ;
- acétate d'éthyle de 2[(1,1-diméthyl-4-méthylsulfanyl-4-oxo-but-2-ynyl)-méthylamino] ;
- 2,5,10,11,11-pentaméthyl-6-oxo-7-oxa-2,5,10-triazatétradéc-12-yne-14-thioate de S-méthyle ;
- 4-[2-(méthoxyméthyl)pyrrolidin-1-yl]-4-méthylpent-2-ynethioate de S-méthyle ;
- 4-(3-Méthoxypyrrolidin-1-yl)-4-méthylpent-2-ynethioate de S-méthyle ;
- 4-méthyl-4-[méthyl(2-phénoxycyclopentyl)amino]pent-2-ynethioate de S-méthyle ;
- 4-(2-((benzyloxy)méthyl)pyrrolidin-1-yl)-4-méthylpent-2-ynethioate de (S)-S-méthyle ;
- 4-[3(benzyloxy)-1 pyrrolidinyl])-4-méthylpent-2-ynethioate de S-méthyle ou ses sels pharmaceutiquement acceptables ou ses isomères optiques, racémates, diastéréoisomères, énantiomères ou tautomères.

**7.** Composé selon la revendication 1, dans lequel :

- X représente S ;
- R1 représente un alkyle en $C_1$-$C_7$ linéaire ou ramifié ;

- R2 représente $CHR_5CHR_6OR_4$ ou $(CHR_5)_vOR_4$ ;
- R4 est choisi parmi H, un aryle, un hétéroaryle, un alkyl-aryle en $C_1$-$C_7$ linéaire ou ramifié et un alkyl-hétéroaryle en $C_1$-$C_7$ linéaire ou ramifié ;
lesdits aryle et hétéroaryle étant éventuellement substitués par un ou plusieurs substituants choisis parmi : -COOH, -NRR' et $-NO_2$ ; et
- R et R' identiques, représentent H.

**8.** Processus de préparation d'un composé selon l'une quelconque des revendications 1 à 7, comprenant :

a) la mise en réaction d'un composé de la formule (II) avec un acide organique ou inorganique

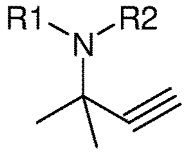

(II)

b) la mise en réaction du composé obtenu à l'étape a) avec une base ;
c) la mise en réaction du composé obtenu à l'étape b) avec du $CO_2$ ;
d) la mise en réaction du composé obtenu à l'étape c) avec du chloroformiate d'alkyle, un réactif pouvant former, avec le composé obtenu à l'étape c), un halogénure d'acide ou un réactif pouvant former, avec le composé obtenu à l'étape c), un anhydride mixte ;
e) la mise en réaction du composé obtenu à l'étape d) avec un composé précurseur d'anion SMe ;

dans lequel R1 et R2 sont définis comme dans l'une quelconque des revendications 1 à 7.

**9.** Composition pharmaceutique comprenant un composé selon l'une quelconque des revendications 1 à 7 et un excipient pharmaceutiquement acceptable.

**10.** Composé selon l'une quelconque des revendications 1 à 7 destiné à être utilisé comme médicament.

**11.** Composé selon l'une quelconque des revendications 1 à 7 destiné à être utilisé dans la prévention ou le traitement du cancer, en particulier de la leucémie.

**12.** Conjugué anticorps-médicament de la formule : B-L-Ab, dans laquelle :

- B est un composé de la formule (I) tel que défini dans la revendication 7 ;
- L est une fraction chimique comprenant une liaison covalente ou une chaîne d'atomes qui attache de manière covalente le Ab au composé de la formule (I) ; et
- Ab est un anticorps choisi parmi : le rituximab, le trastuzumab, l'alemtuzumab, l'ibritumomab, le gemtuzumab, le tiuxétan, le tositumomab, le bévacizumab, le cétuximab, le panitumumab, l'ofatumumab et l'obinutuzumab.

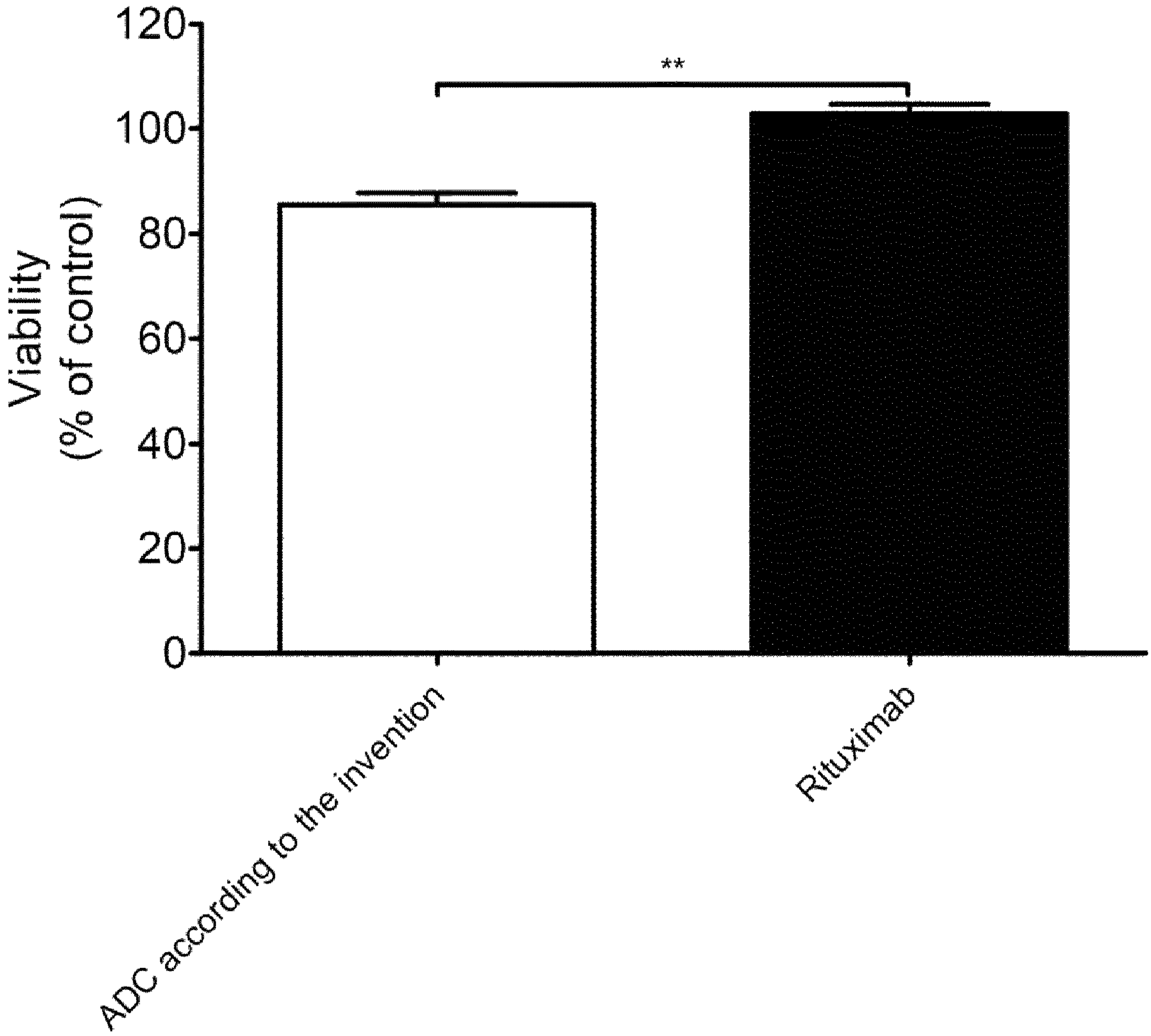
120
100
80
60
40
20
0
Viability
(% of control)
**
ADC according to the invention
Rituximab

FIG.1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20070032476 A
- WO 2017064241 A
- FR 1651283
- EP 2017053457 W
- US 3337625 A, Easton, Nelson R.; Hennion, George F.
- US 19670822 B
- US 3285913 A, Easton, Nelson R.; Hennion, George F.
- WO 2015048363 A
- EP 1296946 A


### Non-patent literature cited in the description


- **QUASH G et al.** Aldehyde dehydrogenase inhibitors: alpha,beta-Acetylenic N-substituted aminothiolesters are reversible growth inhibitors of normal epithelial but irreversible apoptogens for cancer epithelial cells from human prostate in culture. *European Journal Of Medicinal Chemistry,* 2008, vol. 43 (5), 906-916
- Handbook of Chemistry and Physics. CRC Press, Inc, 1995, vol. 1996, 225-226
- Remington's Pharmaceutical Sciences. Mack PublishingCompany, 2000
- **T.W. GREENE ; P. G. M. WUTS.** Protective Groups in Organic Chemistry. John Wiley & Sons Inc, 1999
- **J. F. W. MCOMIE.** Protective Groups in Organic Chemistry. Plenum Press, 1973
- **G.QUASH et al.** *European Journal of Medicinal Chemistry,* 2008, vol. 43, 906-916
- **REMINGTON.** The Science and Practice of Pharmacy. Lippincott Williams & Wilkins, 2000
- **PEREZ et al.** Antibody-drug conjugates: current status and future directions. *Drug Discovery Today,* December 2013
- **MCCOMBS ; SHAWN.** Antibody Drug Conjugates: Design and Selection of Linker, Payload and Conjugation Chemistry. *The AAPS Journal,* March 2015, vol. 17 (2
- *CHEMICAL ABSTRACTS,* 159857-81-5
- *CHEMICAL ABSTRACTS,* 3392-12-9